# EUROPEAN PATENT SPECIFICATION

(11) **EP 3 123 966 B1**
(45) Date of publication and mention of the grant of the patent: **24.04.2019**
(21) Application number: 15768060.4
(22) Date of filing: 26.03.2015
(51) Int. Cl.: A61B 17/34, A61B 1/00, A61B 17/29, A61B 17/00

(54) **ENDOSCOPE AND ENDOSCOPE OPERATIONAL TOOL**
ENDOSKOP UND OPERATIVES ENDOSKOPINSTRUMENT
ENDOSCOPE ET OUTIL OPÉRATIONNEL D'ENDOSCOPE

(30) Priority: 27.03.2014 US 201461971246 P
(43) Date of publication of application: 01.02.2017
(73) Proprietor: Fujifilm Corporation, Minato-ku Tokyo 106-8620 (JP)
(72) Inventor: DEJIMA, Takumi, Ashigarakami-gun Kanagawa 258-8538 (JP)
(74) Representative: Klunker IP Patentanwälte PartG mbB
(86) International application number: PCT/JP2015/059353
(87) International publication number: WO 2015/147157

(56) References cited:
- EP-A1- 2 027 811
- EP-A2- 2 163 217
- EP-A2- 2 238 926
- WO-A1-2005/099558
- WO-A1-2013/176167
- WO-A1-2013/176167
- WO-A2-98/48688
- GB-A- 2 455 343
- JP-A- 2002 209 835
- JP-A- 2005 131 373
- JP-A- 2007 325 721
- JP-A- 2008 006 227
- JP-A- 2010 502 374
- JP-A- 2011 010 671
- JP-A- 2014 014 461
- US-A- 5 373 840
- US-A- 5 913 870

## Description

### BACKGROUND OF THE INVENTION

### 1. Field of the Invention

The present invention relates to an endoscope and particularly, relates to an endoscopic surgical device, an endoscope, and an endoscope operating tool that can operate an endoscope and a treatment tool inserted through two insertion passages provided in an overtube in an interlocking manner.

### 2. Description of the Related Art

In recent years, since invasion to a patient is small compared to surgery in which a laparotomy, a thoracotomy, or the like, is performed, endoscopic surgery using endoscopes (hard endoscopes), such as a laparoscope, has been widely performed. In endoscopic surgery, a plurality of holes are made in a patient's body wall, an endoscope is inserted into a body cavity from one hole of these, and a treatment tool is inserted into the body cavity from another hole. Then, treatment of a living body tissue is performed with the treatment tool while observing the living body tissue within the body cavity with the endoscope.

Generally, in endoscopic surgery, one or a plurality of treatment tools are used simultaneously with the endoscope. Therefore, since it is difficult for one surgeon to simultaneously operate the endoscope and the plurality of treatment tools, for example, a task, such as operating a treatment tool that the surgeon holds with his/her both hands while making an assistant called an endoscopic technician operate the endoscope is normally performed.

Meanwhile, a technique in which the endoscope and the treatment tool can be operated with single hand is disclosed in JP1987-292146A (JP-S62-292146A). Specifically, in an endoscope for ultrasonic treatment in which an ultrasonic treatment tool is incorporated into an endoscope, a working element provided integrally with the endoscope is provided with a front finger hooking part, and the ultrasonic treatment tool is provided with a back finger hooking part. According to this configuration, the forward and backward movement operation of the ultrasonic treatment tool can be performed with a hand holding the endoscope by hooking four fingers other than the thumb of one hand on the front finger hooking part, and hooking the thumb on the back finger hooking GB 2455343 A discloses a grip for an endoscope and EP 2027811 A1 discloses a hooking part to hook a finger of a hand.

### SUMMARY OF THE INVENTION

Meanwhile, in endoscopic surgery, as described above, it is usual that the surgeon's hands are occupied by the operation of the treatment tools, and the operation of the endoscope is performed by the assistant. Therefore, in a case where the observation position of the endoscope is changed, the surgeon needs to give sequential instructions to the assistant. Hence, the task of correctly directing the orientation of the endoscope to a direction desired by the surgeon is difficult, and stress is likely to be imposed on the surgeon. Additionally, since the assistant performs an operation after the surgeon issues an instruction, there is a tendency that surgery time is likely to be prolonged. Additionally, the assistant needs to operate the endoscope so as not to interfere with a surgeon's procedure, and the operation is likely to become complicated.

The present inventor intensively investigated solving such problems, and obtained the knowledge that an overtube inserted into a body cavity being providing with two insertion passages extending in an axial direction, the endoscope being inserted into one insertion passage, the treatment tool is inserted into the other insertion passage, and means capable of moving the endoscope forward and backward in an interlocking manner with the forward and backward movement of the treatment tool are effective. Accordingly, the number of holes made in the patient's body wall can be reduced, the invasion to the patient can be reduced, and the visual field of the endoscope can be easily changed while a surgeon operates the treatment tool without asking for an assistant's help.

However, in a case where the above means is embodied, the necessity for preventing various kinds of operational problems from occurring has newly occurred. For example, when treatment of a living body tissue is being performed, the operation of performing change to a visual field suitable for detailed observation and treatment may be required with the position of the treatment tool being maintained. However, in a configuration in which the forward and backward movement of the endoscope is made possible in an interlocking manner with the forward and backward movement of the treatment tool, it is difficult to perform the operation of changing the visual field of the endoscope without both the surgeon's hands being released from the treatment tool during treatment.

Additionally, in the configuration in which the forward and backward movement of an endoscope is made possible in an interlocking manner with the forward and backward movement of the treatment tool, it is required from a viewpoint of low invasion to a patient that, while preventing any interference between the endoscope and the treatment tool inserted through the two insertion passages provided in the overtube, the external diameter of the overtube is made small by bringing these insertion passages as close to each other as possible.

In contrast, in the technique disclosed in JP1987-292146A (JP-S62-292146A), the forward and backward movement operation of the treatment tool can be performed by the hand holding the endoscope. However, this technique does not have the configuration in which the endoscope and the treatment tool are inserted into one overtube, and does not have the technical idea of changing the visual field of the endoscope with the position of the treatment tool being maintained while preventing any interference between the endoscope and the treatment tool.

The invention has been made in view of such circumstances, and an object thereof is to provide an endoscope adapted to be used for an endoscopic surgical device, and an endoscope operating tool that can change to a visual field suitable for detailed observation and treatment and can improve surgical efficiency, in a configuration in which the endoscope and a treatment tool inserted through two insertion passages provided in an overtube are operable in an interlocking manner with each other.

In order to achieve the above object, an endoscope according to claim 1 is provided. Further embodiments of the invention are provided in the dependent claims.

According to the invention, the hooking part is provided in the flexible cable connected to the base end of the endoscope insertion part. Thus, a surgeon can hook a finger of his/her hand operating the treatment tool on the hooking part to perform the forward and backward movement operation of the endoscope, without disengaging his/her hand from the treatment tool while preventing any interference between the endoscope and the treatment tool. Accordingly, it is possible to perform change to a visual field suitable for detailed observation and treatment, and surgical efficiency can be improved.

In the endoscope of the invention, an aspect in which the hooking part includes an opening formed in a ring shape or in a C-shape, and the endoscope insertion part is moved forward and backward by passing the finger through the opening is preferable.

According to this aspect, by passing the finger of the hand operating the treatment tool through the opening of the hooking part, the cable of the endoscope provided with the hooking part can be constrained to being on the operating part side of the treatment tool, and it is possible to perform the forward and backward movement operation of the endoscope in a stable state.

In the endoscope of the invention, an aspect in which the hooking part is made of an elastic body is preferable.

According to this aspect, the resistance caused when the hooking part interferes with the operating part of the treatment tool can be reduced, and the operation of the hooking part can be prevented from becoming difficult to perform.

In the endoscope of the invention, an aspect in which a plurality of hooking parts are provided in an axial direction of the cable is preferable.

According to this aspect, by hooking the finger of the hand operating the treatment tool on any one of the plurality of hooking parts, operation becomes possible without influence from the positional relationship between the endoscope and the treatment tool.

In the endoscopic surgical device related to the aspect of the invention, an aspect in which the hooking part is provided so as to be rotatable around an axis of the cable is preferable.

According to this aspect, since the hooking part is configured so as to be rotatable around the axis of the cable, the hooking part can be directed to a direction in which operation is easy irrespective of the rotational position of the endoscope with respect to the overtube.

In the endoscope of the invention, an aspect in which the hooking part is provided so as to be attachable to and detachable from the cable is preferable.

According to this aspect, by detaching the hooking part from the cable, the hooking part does not become obstructive in a case where the endoscope is used alone, and cleaning and sterilization of mounting portions of the hooking part can be easily performed.

In the endoscopic surgical device related to an aspect of the invention, an aspect in which an interlocking member including an endoscope-coupled part coupled to the endoscope insertion part inserted through the endoscope insertion passage and a treatment tool-coupled part coupled to the treatment tool insertion part inserted through the treatment tool insertion passage and being arranged inside the overtube body so as to be movable forward and backward is further included is preferable. Here, the interlocking member includes a dead zone where the forward and backward movement of either the endoscope insertion part or the treatment tool insertion part does not interlock with the movement of the other and a sensing zone where the forward and backward movement of either the endoscope insertion part or the treatment tool insertion part interlocks with the movement of the other.

According to this aspect, the endoscope moves forward and backward with play with respect to the forward and backward movement of the treatment tool. Thus, in a case where the treatment tool has been minutely displaced in the axial direction, the range of the object to be observed does not change, that is, the size of the object to be observed can be prevented from fluctuating. Accordingly, a sense of perspective can be suitably maintained, and a stable observation image in which the amount of forward and backward movement of the treatment tool can be recognized can be provided. Additionally, in a case where the treatment tool has been greatly displaced in the axial direction, the range of an observation image obtained by the endoscope is changed in an interlocking manner with this large displacement. Thus, since the size of the object to be observed changes according to the operation of the treatment tool, it is possible to obtain an image desired by a surgeon simply, and operability can be improved.

In the endoscope of the invention, an aspect in which the endoscope insertion part is hard is preferable.

According to the invention, it is possible to change a visual field suitable for detailed observation and treatment and surgical efficiency can be improved, in a configuration in which the endoscope and the treatment tool inserted through the two insertion passages provided in the overtube are operable in an interlocking manner with each other.

### BRIEF DESCRIPTION OF THE DRAWINGS

Fig. 1 is a schematic block diagram of an endoscopic surgical device related to the invention.
Fig. 2 is a plan view illustrating a distal end surface of an endoscope insertion part.
Fig. 3 is an external perspective view illustrating an overtube.
Fig. 4 is a sectional view illustrating the internal structure of the overtube.
Fig. 5 is an enlarged sectional view illustrating a portion of Fig. 4 in an enlarged manner.
Fig. 6 is a sectional view when viewed from arrow 6-6 in Fig. 5.
Fig. 7 is an explanatory view used for the description of the action of the slider.
Fig. 8 is an explanatory view used for the description of the action of the slider.
Fig. 9 is an explanatory view used for the description of the action of the slider.
Fig. 10 is an enlarged appearance view illustrating a forward and backward movement operating part in a flexible part of an endoscope.
Fig. 11 is a perspective view illustrating a state where a hooking part of the forward and backward movement operating part of the endoscope is detached from the flexible part.
Fig. 12 is a view illustrating an aspect in which an operating part of a treatment tool is gripped.
Fig. 13 is a view illustrating an aspect in which the forward and backward movement operating part of the endoscope is operated by an index finger of a right hand gripping the operating part of the treatment tool.
Figs. 14A to 14C are views illustrating an aspect of the operation when only the treatment tool moves forward and backward.
Figs. 15A to 15C are views illustrating an aspect of the operation when only the endoscope moves forward and backward.
Figs. 16A to 16C are views illustrating an aspect of the operation when the endoscope moves forward and backward in an interlocking manner with the treatment tool through an interlocking function of a slider.
Figs. 17A to 17C are views illustrating an aspect of the operation when the endoscope moves forward and backward in an interlocking manner with the treatment tool without resort to the interlocking function of the slider.
Fig. 18 is a view illustrating another form of the forward and backward movement operating part of the endoscope.
Fig. 19 is a view illustrating still another form of the forward and backward movement operating part of the endoscope.
Fig. 20 is a view illustrating yet still another form of the forward and backward movement operating part of the endoscope.
Fig. 21 is a flowchart illustrating a procedure when an overtube is inserted into a patient's body wall.
Figs. 22A to 22C are perspective views illustrating the overtube, an oversheath, and an inner needle.
Figs. 23A and 23B are views used for the description when the oversheath is mounted on the overtube.
Figs. 24A and 24B are views used for the description when the inner needle is mounted on the overtube.
Figs. 25A and 25B are views used for the description when the endoscope is mounted on a treatment-tool-side shaft part of the inner needle.
Fig. 26 is a sectional view illustrating the configuration of a distal end of the treatment-tool-side shaft part of the inner needle.
Fig. 27 is a view used for the description when a pneumoperitoneum tube is mounted on the oversheath.
Figs. 28A and 28B are views used for the description when the overtube is arranged at a body wall.
Fig. 29 is a view used for the description when pneumoperitoneum is performed on a body cavity.
Fig. 30 is a view illustrating an aspect in which the inner needle is extracted from the overtube.
Fig. 31 is a view illustrating an aspect in which the endoscope and the treatment tool are mounted on the overtube.

### DESCRIPTION OF THE PREFERRED EMBODIMENTS

Preferred embodiments of the invention will be described below in detail according to the accompanying drawings. In addition, any of the drawings may illustrate main parts in an exaggerated manner for description, and may have dimensions different from actual dimensions.

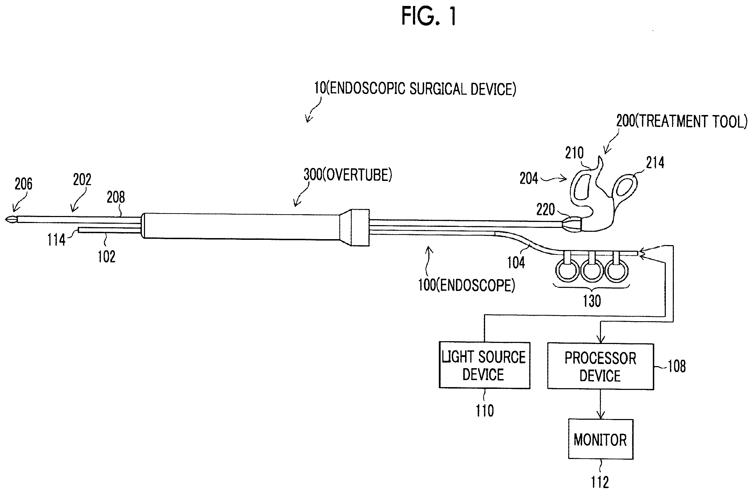

Fig. 1 is a schematic block diagram of an endoscopic surgical device related to the invention. As illustrated in Fig. 1, an endoscopic surgical device 10 includes an endoscope 100 that observes the inside of a patient's body cavity, a treatment tool 200 for examining or treating a diseased site within the patient's body cavity, and an overtube 300 that is inserted into a body wall and guides the endoscope 100 and the treatment tool 200 into the body cavity.

The endoscope 100 is, for example, a hard endoscope, such as a laparoscope, and includes an insertion part 102 (hereinafter referred to as "endoscope insertion part") that is inserted into a body cavity and has an outer peripheral part surrounded by an elongated hard tubular body, and a cable part 104 that is provided continuously with a base end side of the endoscope insertion part 102 and that has an outer peripheral part surrounded by an elongated flexible tubular body.

The cable part 104 indicates a flexible cable portion in which a wire rod, such as a cable or a light guide, which extends from a base end of the endoscope insertion part 102, is housed by covering the wire rod with, for example, a flexible insulating member, such as polyvinyl chloride.

A connector (not illustrated) is provided at an end of the cable part 104 on its extension destination, and each of a processor device 108 and a light source device 110 is detachably connected to the cable part via the connector. Additionally, the processor device 108 is connected to a monitor 112 via a cable.

In addition, the cable part 104 is provided with a forward and backward movement operating part 130 for hooking an index finger of a right hand gripping an operating part 204 of the treatment tool 200, and performing a forward and backward movement operation of the endoscope 100 in a forward-backward direction. The forward and backward movement operating part 130 will be described below in detail.

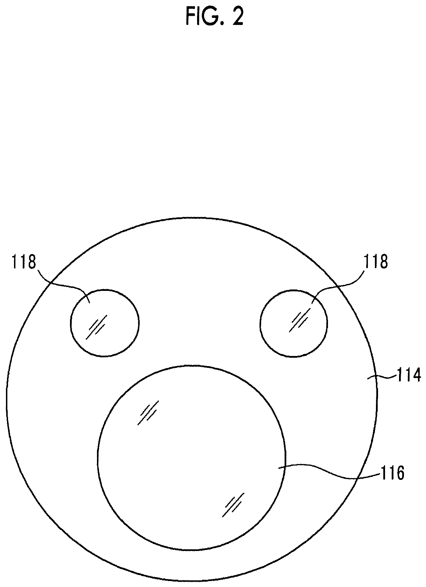

As illustrated in Fig. 2, a distal end surface 114 of the endoscope insertion part 102 is provided with an observation window 116 and illumination windows 118 and 118.

The observation window 116 is a constituent element of an observation part of the endoscope 100, and an objective lens of an observation optical system, and an image pick-up element, such as a charge coupled device (CCD) or a complementary metal oxide semiconductor (CMOS), which is arranged at an image pick-up position of the objective lens, are disposed behind the observation window 116. A signal cable (not illustrated) connected to an image pick-up element of this observation part is inserted through the endoscope insertion part 102 and the cable part 104 of Fig. 1, is provided to extend up to the connector (not illustrated), and is connected to the processor device 108. An observation image picked up from the observation window 116 is formed on a light-receiving surface of the image pick-up element, and is converted into electrical signals (image pick-up signals), and the electrical signals are output to the processor device 108 via the signal cable and are converted into video signals. Then, the video signals are output to the monitor 112 connected to the processor device 108, and the observation image (endoscope image) is displayed on a screen of the monitor 112.

An exit end of the light guide (not illustrated) is disposed behind the illumination windows 118 and 118 of Fig. 2. The light guide is inserted through the endoscope insertion part 102 and the cable part 104 of Fig. 1 and has an incident end disposed within the connector (not illustrated). Therefore, by coupling the connector to the light source device 110, the illumination light radiated from the light source device 110 is transmitted to the illumination windows 118 and 118 via the light guide, and is radiated forward from the illumination windows 118 and 118. In addition, in Fig. 2, the two illumination windows 118 and 118 are disposed on the distal end surface 114 of the endoscope insertion part 102. However, the number of illumination windows 118 is not limited, and the number thereof may be one or may be three or more.

As illustrated in Fig. 1, the treatment tool 200 consists of, for example, forceps, and includes an elongated insertion part 202 (hereinafter referred to as a "treatment tool insertion part") that is inserted into a body cavity, an operating part 204 that is provided on the base end side of the treatment tool insertion part 202 and is gripped by a surgeon, and a treatment part 206 that is provided on a distal end side of the treatment tool insertion part 202 and is operable by the operation of the operating part 204.

The treatment tool insertion part 202 is provided with a tubular sheath 208, and an operating shaft (not illustrated) that is inserted into the sheath 208 so as to be movable in the direction of an axial center. Moreover, the operating part 204 is provided with a fixed handle 210, and a movable handle 214 that is turnably coupled to the fixed handle 210 via a turning pin. A base end of the operating shaft is coupled to the movable handle 214.

The treatment part 206 is provided with a pair of gripping members that is openable and closable. The gripping members are coupled to a distal end of the operating shaft via a driving mechanism (not illustrated). With the turning operation of the movable handle 214 of the operating part 204, the gripping members of the treatment part 206 are opened and closed via the operating shaft and the driving mechanism.

Additionally, a rotating handle 220 is provided at a base end of the treatment tool insertion part 202 and a distal end of the operating part 204 so as to be turnable around the axis of the treatment tool insertion part 202 (sheath 208). If the rotating handle 220 is rotated, the operating shaft rotates around the axis of the sheath 208, and the pair of gripping members of the treatment part 206 rotates around the axis of the sheath 208 in its entirety via the operating shaft and the driving mechanism.

In addition, the treatment tool 200 is not limited to the forceps, and may be, for example, other treatment tools, such as a laser probe, a suture device, an electric scalpel, a needle holder, and an ultrasonic aspirator.

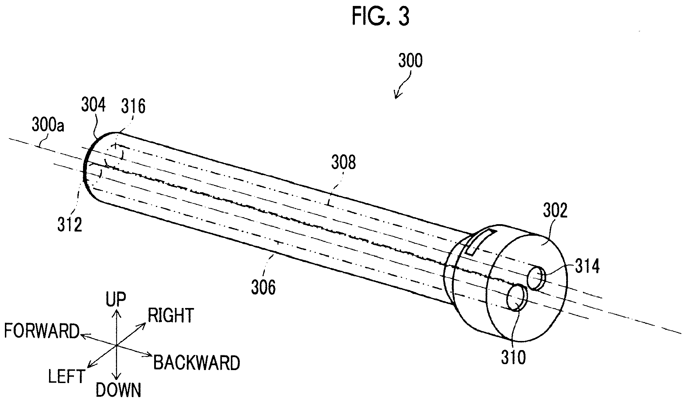

Fig. 3 is an external perspective view illustrating the overtube 300.

As illustrated in this drawing, the overtube 300 has an elongated columnar shape as a whole, and has an endoscope insertion passage 306 through which the endoscope insertion part 102 of the endoscope 100 is inserted so as to be movable forward and backward, and a treatment tool insertion passage 308 through which the treatment tool insertion part 202 of the treatment tool 200 is inserted so as to be movable forward and backward. These insertion passages are parallel to a reference axis 300a (longitudinal axis) indicating a central axis of the overtube.

In addition, regarding the position and orientation of a space where the overtube 300 has been arranged, terms called forward, backward, left, right, up, and down are used with the orientation from the base end surface 302 in a direction along the reference axis 300a to the distal end surface 304 defined as the forward and with the orientation from the reference axis 300a to the endoscope insertion passage 306 defined as the left.

The base end surface 302 of the overtube 300 is provided with an endoscope insertion port 310 that allows the endoscope insertion part 102 to be inserted into the endoscope insertion passage 306 therethrough, and a treatment tool insertion port 314 that allows the treatment tool insertion part 202 to be inserted into the treatment tool insertion passage 308 therethrough.

The distal end surface 304 of the overtube 300 is provided with an endoscope delivery port 312 that allows the endoscope insertion part 102 inserted into the endoscope insertion passage 306 to be delivered to the outside therethrough, and a treatment tool delivery port 316 that allows the treatment tool insertion part 202 inserted into the treatment tool insertion passage 308 to be delivered to the outside therethrough.

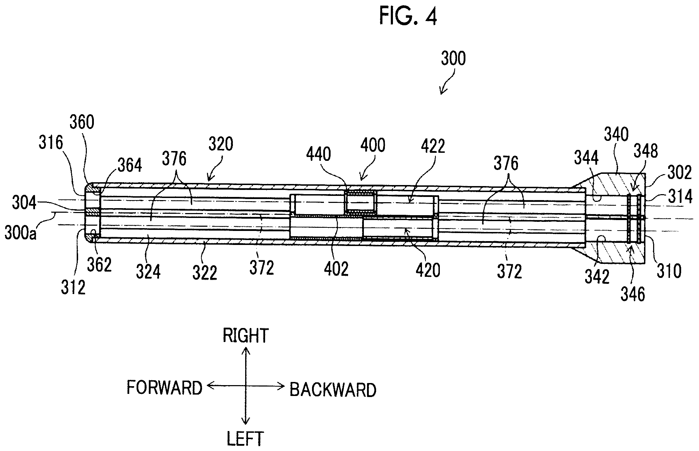

Fig. 4 is a sectional view illustrating the internal structure of the overtube 300, and illustrates a section cut in a plane that includes the reference axis 300a and is orthogonal to an upward-downward direction.

As illustrated in this drawing, the overtube 300 has an overtube body 320 that occupies substantially the entire area in the forward-backward direction, a base end cap 340 that is attached to a rear end (base end) of the overtube 300, a distal end cap 360 that is attached to a distal end, and a slider 400 (the slider 400 is one form of an interlocking member) that is arranged inside the overtube 300.

The overtube body 320 is formed in an elongated cylindrical shape having the reference axis 300a as a central axis using hard resins, metals, or the like, and has an outer wall 322 that surrounds an outer periphery, and a cavity part 324 that penetrates from a base end of the overtube body 320 to a distal end thereof.

The cavity part 324 includes spaces serving as the endoscope insertion passage 306 and the treatment tool insertion passage 308, and houses the slider 400 and the like.

The base end cap 340 is formed in a columnar shape of which the diameter is made larger than the external diameter of the overtube body 320 using hard resins, metals, or the like, and a rear end surface thereof constitutes the base end surface 302 of the overtube 300. The base end cap 340 is provided with a through-hole 342 and a through-hole 344 that form a portion of the endoscope insertion passage 306 and a portion of the treatment tool insertion passage 308, respectively. In the base end surface 302, an opening of the through-hole 342 is equivalent to the above-described endoscope insertion port 310, and an opening of the through-hole 344 is equivalent to the above-described treatment tool insertion port 314.

Additionally, the through-holes 342 and 344 are provided with valve members 346 and 348. The valve members 346 and 348, for example, open in a case where the endoscope insertion part 102 and the treatment tool insertion part 202 are inserted therethrough and come into close contact with outer peripheral surfaces (side surfaces) of the endoscope insertion part 102 and the treatment tool insertion part 202 without a substantial gap. This secures the airtightness of spaces closer to the distal end side than the valve members 346 and 348, and reduces the leakage or the like of a pneumoperitoneum gas injected into the body cavity to the outside of the body.

The distal end cap 360 is formed of hard resins, metals, or the like, and a front end surface thereof constitutes the distal end surface 304 of the overtube 300. The distal end cap 360 is provided with a through-hole 362 and a through-hole 364 that form a portion of the endoscope insertion passage 306 and a portion of the treatment tool insertion passage 308, respectively. In the distal end surface 304, an opening of the through-hole 362 is equivalent to the above-described endoscope delivery port 312, and an opening of the through-hole 364 is equivalent to the treatment tool delivery port 316..

The above base end cap 340 and the above distal end cap 360 are some of the constituent elements of the overtube body of the invention, and may be formed separately from or formed integrally with the overtube body 320.

The slider 400 is housed within the cavity part 324 of the overtube body 320, and is supported so as to be movable forward and backward in the direction of the reference axis 300a. The slider 400 is an interlocking member that is coupled to the endoscope insertion part 102 inserted through the endoscope insertion passage 306 and the treatment tool insertion part 202 inserted through the treatment tool insertion passage 308 and that has a dead zone where the forward and backward movement of either the endoscope insertion part or the treatment tool insertion part in the forward-backward direction (axial direction) does not interlock with the movement of the other and a sensing zone where the forward and backward movement of either the endoscope insertion part or the treatment tool insertion part interlocks with the movement of the other. That is, the endoscope insertion part 102 is adapted to interlock with the forward and backward movement of the treatment tool insertion part 202 in the axial direction with play by the slider 400.

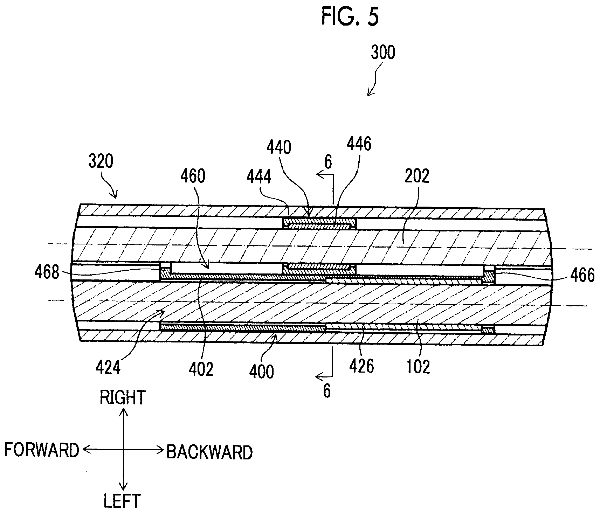

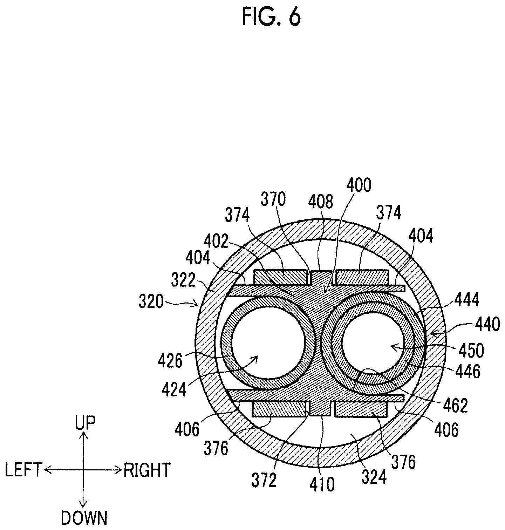

Fig. 5 is an enlarged sectional view illustrating a portion, in which the slider 400 is arranged in Fig. 4, in an enlarged manner, and illustrates a state where the endoscope insertion part 102 and the treatment tool insertion part 202 have been inserted through the endoscope insertion passage 306 and the treatment tool insertion passage 308, respectively. Fig. 6 is a sectional view when viewed from arrow 6-6 in Fig. 5.

As illustrated in Figs. 5 and 6, the slider 400 has a slider body 402 (slider member) that holds components of the slider 400. As illustrated in Fig. 6, protruding strips 408 and 410 that extend in the direction (forward-backward direction) of the reference axis 300a are formed on a flat upper surface 404 and a flat lower surface 406 of the slider body 402.

Meanwhile, a pair of left and right long plate-shaped guide plates 374 and 374 and a pair of left and right long plate-shaped guide plates 376 and 376, which are laid between the base end cap 340 and the distal end cap 360, are respectively supported by an upper part and a lower part within the cavity part 324 of the overtube body 320, and guide grooves 370 and 372, which extend in the direction of the reference axis 300a from the base end cap 340 to the distal end cap 360, are formed by a gap between the guide plates 374 and 374 and a gap between the guide plates 376 and 376.

The protruding strips 408 and 410 of the slider body 402 are respectively fitted into the guide grooves 370 and 372 within the cavity part 324, and the upper surface 404 and the lower surface 406 are arranged in a state where these surfaces have contacted or approached the guide plates 374 and 374 and the guide plates 376 and 376..

Accordingly, the slider 400 is supported so as to be movable forward and backward in the forward-backward direction within the cavity part 324, and is supported in a state where the movement of the slider in the upward-downward direction and in the leftward-rightward direction and the rotation of the slider in all directions are restricted (a state where the rotation of the slider around at least the reference axis 300a is impossible). Additionally, the slider 400 moves forward and backward within a movable range having a position where the slider abuts against the base end cap 340 as a rear end, and having a position where the slider abuts against the distal end cap 360 as a front end.

In addition, the guide grooves 370 and 372 may not be formed by the guide plates 374 and 374 and the guide plates 376 and 376 arranged within the cavity part 324 of the overtube body 320, and may be formed in the outer wall 322 of the overtube body 320 or may be formed by other configurations.

Additionally, the slider 400, as illustrated in Fig. 4, has an endoscope-coupled part 420 that is coupled (engaged) with the endoscope insertion part 102, and a treatment tool-coupled part 422 that is coupled (engaged) with the treatment tool insertion part 202.

The endoscope-coupled part 420 is provided on the left side of the slider body 402, and includes a through-hole 424 (refer to Fig. 6) in which a space serving as the endoscope insertion passage 306 is secured within the cavity part 324 of the overtube body 320 and through which, as illustrated in Fig. 5, the endoscope insertion part 102 is inserted, and a pressure-contact member 426 that is fixed to the through-hole 424, is brought into pressure contact with the outer peripheral surface (side surface) of the endoscope insertion part 102 inserted through the endoscope insertion passage 306. The pressure-contact member 426 is annularly formed of elastic materials, such as elastic rubber, as illustrated in Fig. 6.

Accordingly, when the endoscope insertion part 102 has been inserted through the endoscope insertion passage 306, as illustrated in Fig. 5, the endoscope insertion part 102 is inserted through the through-hole 424, and the pressure-contact member 426 is brought into pressure contact with (engaged with) the outer peripheral surface of the endoscope insertion part 102. The endoscope insertion part 102 and the slider 400 (slider body 402) are coupled (engaged) with each other in an interlockable manner via the pressure-contact member 426, and the slider 400 (slider body 402) also integrally moves forward and backward in an interlocking manner with the forward and backward movement of the endoscope insertion part 102 in the forward-backward direction (axial direction).

In addition, since the coupling herein is based on the elastic force of the pressure-contact member 426, the engagement position (the position of the endoscope insertion part 102 where the slider 400 is engaged) of the endoscope insertion part 102 coupled to the slider 400 (slider body 402) can be arbitrarily adjusted.

The treatment tool-coupled part 422, as illustrated in Fig. 4, is provided on the right side of the slider body 402, and as illustrated in Fig. 5, includes a sleeve 440 (sleeve member) that is coupled to the treatment tool insertion part 202, and a guide part 460 that guides the sleeve 440 so as to be movable forward and backward in the forward-backward direction.

The sleeve 440, as illustrated in Fig. 6, includes a sleeve body (frame body) 444 formed in a cylindrical shape, and a pressure-contact member 446 fixed to the inside of the sleeve body 444. The pressure-contact member 446 is annularly formed of elastic materials, such as elastic rubber.

Accordingly, when the treatment tool insertion part 202 has been inserted through the treatment tool insertion passage 308, as illustrated in Fig. 5, the treatment tool insertion part 202 is inserted through the inside (the through-hole 450 of Fig. 6) of the pressure-contact member 446, the pressure-contact member 446 is brought into pressure contact with (engaged with) the outer peripheral surface of the treatment tool insertion part 202. The treatment tool insertion part 202 and the sleeve 440 are coupled with each other in an interlockable manner via the pressure-contact member 446, and the sleeve 440 also integrally moves forward and backward in an interlocking manner with the forward and backward movement of the treatment tool insertion part 202 in the forward-backward direction (axial direction).

Additionally, the sleeve 440 also rotates with respect to the slider body 402 in an interlocking manner with the rotation of the treatment tool insertion part 202 around the axis thereof.

In addition, since the coupling between the treatment tool insertion part 202 and the sleeve 440 herein is based on the elastic force of the pressure-contact member 446, the engagement position (the position of the treatment tool insertion part 202 where the sleeve 440 is engaged) of the treatment tool insertion part 202 coupled to the sleeve 440 can be arbitrarily adjusted.

Meanwhile, the guide part 460 of the treatment tool-coupled part 422, as illustrated in Fig. 6, is formed by a space surrounded by a guide surface 462 of the slider body 402 that extends in the direction of the reference axis 300a within the cavity part 324 of the overtube body 320, and an inner peripheral surface of the overtube body 320. The sleeve 440 is housed and arranged in the space of the guide part 460, is supported so as to be movable in the forward-backward direction and rotatable around its axis, and is supported in a state where the movement of the sleeve in the upward-downward direction and in the leftward-rightward direction is restricted.

Additionally, the guide part 460 is provided so as to fall within a range from a base end of the slider body 402 to a distal end thereof, and as illustrated in Fig. 5, has end edge parts 466 and 468, which are formed to protrude in a direction orthogonal to the guide surface 462 along an end edge of the guide surface 462, respectively, on the base end side and the distal end side of the slider body 402.

The end edge parts 466 and 468 abut against the end of the sleeve 440 to restrict the movement of the sleeve 440, when the sleeve 440 arranged in the space of the guide part 460 moves forward and backward in the forward-backward direction.

Therefore, the sleeve 440 moves forward and backward within a movable range having a position where the sleeve abuts against the end edge part 466 as a rear end, and having a position where the sleeve abuts against the end edge part 468 as a front end. However, the rear end and the front end of the movable range of the sleeve 440 may not be restricted by the end edge part 466 and the end edge part 468.

According to the slider 400 configured as described above, the endoscope insertion part 102 inserted through the endoscope insertion passage 306 of the overtube 300 and the slider body 402 are coupled together, and the treatment tool insertion part 202 inserted through the treatment tool insertion passage 308 of the overtube 300 and the sleeve 440 are coupled together.

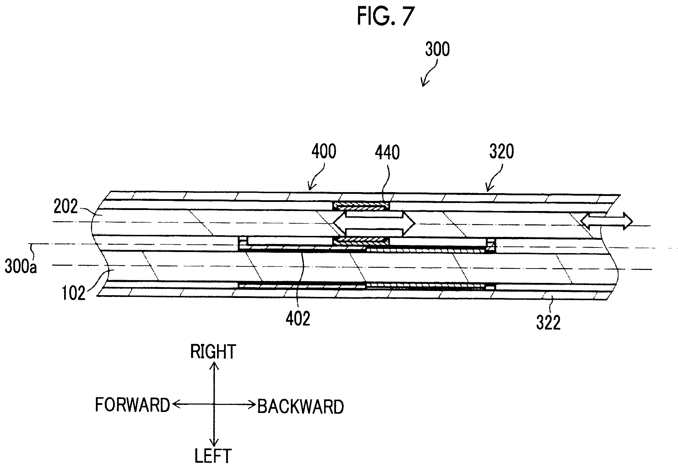

As illustrated in Fig. 7, it is supposed that a surgeon performs a forward and backward movement operation for moving the treatment tool insertion part 202 forward and backward in the axial direction (forward-backward direction) in a state where the sleeve 440 has not reached the rear end and the front end of the movable range thereof with respect to the slider body 402 (guide part 460).

In this case, in a case where the sleeve 440 has moved forward and backward within the movable range thereof with respect to the slider body 402, the slider body 402 does not move with respect to the forward and backward movement of the treatment tool insertion part 202. Therefore, the dead zone where the endoscope insertion part 102 does not interlock with the forward and backward movement of the treatment tool insertion part 202 is present.

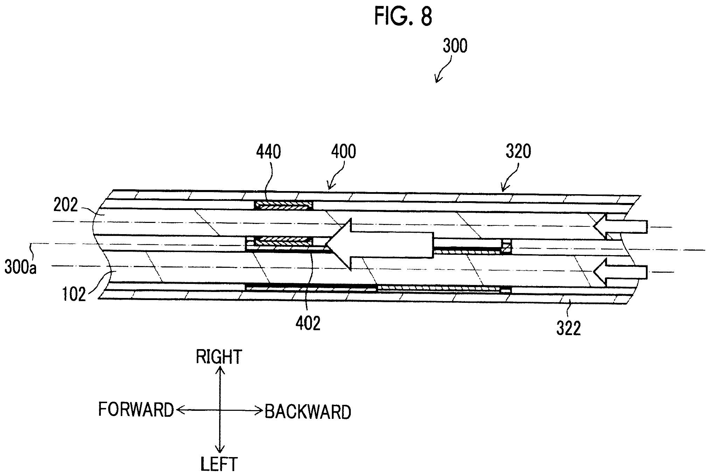

Meanwhile, as illustrated in Fig. 8, if the treatment tool insertion part 202 is operated to move forward in a state where the sleeve 440 reaches the front end of the movable range thereof with respect to the slider body 402, the sleeve 440 and the slider body 402 move forward with respect to the overtube body 320 together with the treatment tool insertion part 202. Accordingly, the endoscope insertion part 102 moves forward in an interlocking manner with the treatment tool insertion part 202.

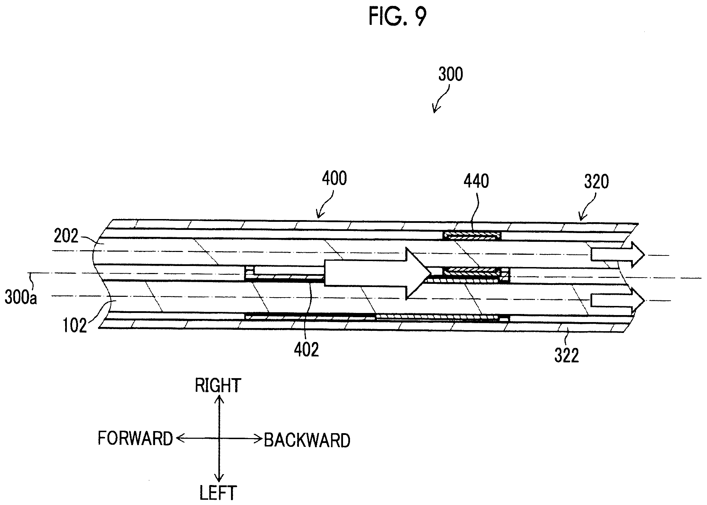

Similarly, as illustrated in Fig. 9, if the treatment tool insertion part 202 is operated to move backward in a state where the sleeve 440 reaches the rear end of the movable range thereof with respect to the slider body 402, the sleeve 440 and the slider body 402 move backward with respect to the overtube body 320 together with the treatment tool insertion part 202. Accordingly, the endoscope insertion part 102 moves backward in an interlocking manner with the treatment tool insertion part 202.

Therefore, in a case where the treatment tool insertion part 202 has been greatly displaced in the axial direction as described above (in a case where a large amplitude of forward and backward movement has been performed), the endoscope insertion part 102 is displaced in the axial direction in an interlocking manner with the treatment tool insertion part 202, and in a case where the displacement of the treatment tool insertion part 202 in the axial direction is small (in a case where a small amplitude of forward and backward movement is performed), the endoscope insertion part 102 is not displaced in the axial direction.

Accordingly, in a case where the displacement of the treatment tool insertion part 202 in the axial direction is large (in a case where a large amplitude of forward and backward movement has been performed) when a surgeon has moved the treatment tool insertion part 202 forward and backward in the axial direction, the endoscope insertion part 102 also moves in an interlocking manner forward, backward, up, down, right, and left. Thus, the visual field, orientation, and the like of the endoscope 100 can be changed as intended by a surgeon. Additionally, the visual field is always given to pick up an image of the distal end of the treatment tool and consequently, an image that is optimal for treatment is automatically provided. In a case where it is desired to check sites other than a site to be treated, the checking can be performed by moving the treatment tool insertion part 202, and a surgeon can perform operations as desired. Therefore, an assistant (endoscopic technician) who operates the endoscope 100 apart from the surgeon can be made unnecessary, and a troublesome condition in which the surgeon should instruct an assistant about the visual field, orientation, and the like of the endoscope serially can be eliminated.

Additionally, in a case where the displacement of the treatment tool insertion part 202 in the axial direction is small (in a case where a small amplitude of forward and backward movement has been performed), the endoscope insertion part 102 does not interlock. Therefore, the size of an object to be observed within an observation image can be prevented from fluctuating unnecessarily, a sense of perspective can be suitably maintained, and a stable observation image can be provided.

Next, the forward and backward movement operating part 130 provided in the cable part 104 of the endoscope 100 illustrated in Fig. 1 will be described.

As illustrated in Fig. 1, the forward and backward movement operating part 130 is arranged at a position adjacent to the operating part 204 of the treatment tool 200, in a state where the endoscope 100 and the treatment tool 200 are respectively inserted through the endoscope insertion passage 306 and the treatment tool insertion passage 308 of the overtube 300, and the position of the distal end surface 114 of the endoscope 100 in the axial direction (forward-backward direction) is adjusted with respect to the position of the treatment part 206 of the treatment tool 200 so that the treatment part 206 is reflected on an observation image.

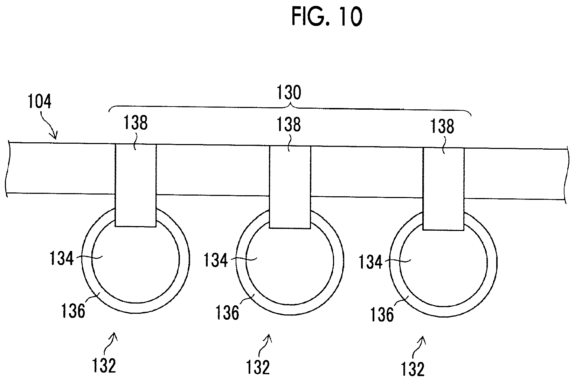

Fig. 10 is an enlarged appearance view illustrating the forward and backward movement operating part 130 in the cable part 104 of the endoscope 100. As illustrated in this drawing, the forward and backward movement operating part 130 has three hooking parts 132 with the same configuration, and the hooking parts 132 are arranged at positions with substantially regular intervals in the axial direction of the cable part 104 of an endoscope 100. In addition, the hooking parts 132 are equivalent to an endoscope operating tool of the invention.

Although each hooking part 132 will be described below in detail, the hooking part has an opening 134 with a size such that an index finger can be passed therethrough.

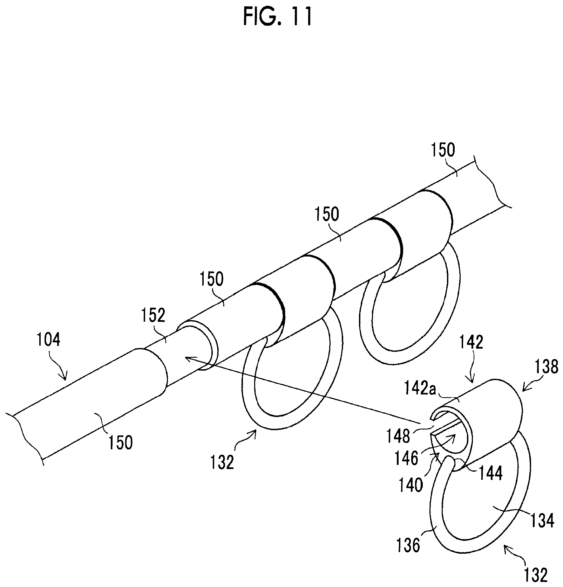

Additionally, each hooking part 132 is an elastic body, is attachable to and detachable from the cable part 104 as illustrated in Fig. 11, and is constituted of a ring member 136 that forms the opening 134, and a coupling member 138 that detachably couples the ring member 136 to the cable part 104.

The ring member 136 is formed in an annular shape (ring shape) using elastic materials (for example, rubber material), and a hole portion inside the ring member forms the above-described opening 134. Additionally, the ring member 136 has a diameter (internal diameter) to such a degree that a finger (for example, an index finger) can be inserted through the opening 134.

The coupling member 138 is formed of the same elastic materials as the ring member 136, and has a holding part 140 and a locking part 142. However, the coupling member 138 may be formed of resin materials.

A through-hole 144 is formed in the holding part 140, and the ring member 136 is inserted through the through-hole 144. Accordingly, the ring member 136 is held by the holding part 140 of the coupling member 138, and is held so as to be rotatable (rockable) in a direction orthogonal to an opening surface of the opening 134 with the portion thereof inserted through the through-hole 144 as a center.

In addition, the ring member 136 may not be endless, and may have a form having two end surfaces where a portion of a circular ring is cut out, and having both ends in the vicinity of the end surfaces supported by the holding part 140 of the coupling member 138. Additionally, the ring member 136 is not necessarily rockable with respect to the holding part 140, and the ring member 136 may be formed integrally with the coupling member 138.

The locking part 142 of the coupling member 138 is constituted of a plate-shaped body 142a that is provided to extend from the holding part 140 and is bent in a circular-arc shape. A locking hole 146 is formed by a columnar cavity portion surrounded by the bent plate-shaped body 142a, and a gap part 148 is formed between the plate-shaped body 142a and the holding part 140.

Meanwhile, the cable part 104 of the endoscope 100 has a larger-diameter part 150 and a smaller-diameter part 152 that are different from each other in diameter, and smaller-diameter parts 152 having a smaller diameter than the larger-diameter part 150 are provided at positions of three places with substantially regular intervals in the axial direction of the cable part 104 as positions where the hooking parts 132 are mounted.

Additionally, the diameter of each smaller-diameter part 152 is slightly larger than the diameter of the locking hole 146 of the coupling member 138 in the hooking part 132, and the axial length of the smaller-diameter part 152 is substantially the same as the width of the locking part 142 (plate-shaped body) of the coupling member 138 in the hooking part 132.

According to this, if the gap part 148 in the coupling member 138 of the hooking part 132 is laid along the smaller-diameter part 152 at a desired position of the cable part 104 and the coupling member 138 is pushed into the smaller-diameter part 152, the plate-shaped body 142a of the locking part 142 is elastically deformed, whereby the smaller-diameter part 152 is inserted through the gap part 148 and enters the locking hole 146. Accordingly, the smaller-diameter part 152 is fitted to the locking hole 146, and an inner peripheral surface of the plate-shaped body 142a of the locking part 142 abuts against an outer peripheral surface of the smaller-diameter part 152 without a substantial gap.

Accordingly, the locking part 142 is restricted by a step between the smaller-diameter part 152 and the larger-diameter part 150 of the cable part 104, and is mounted on the cable part 104 in a state where the movement of the cable part 104 in the axial direction is restricted. Additionally, the locking part 142 is mounted so as to be rotatable around the axis of the cable part 104. Therefore, the hooking part 132 is mounted on the cable part 104 so as to be rotatable around the axis of the cable part 104, in a state where movement of the cable part 104 in the axial direction is restricted.

In addition, by using an elastic body as the overall hooking part 132 or the ring member 136, it is possible to prevent a situation in which resistance is generated and it becomes difficult to perform the operation of the hooking part 132 when there is any interferes with the operating part 204 of the treatment tool 200.

Additionally, since the hooking part 132 is rotatable around the axis of the cable part 104, the hooking part 132 can be directed to a direction in which operation is easy irrespective of the rotational position of the endoscope 100 with respect to the overtube 300.

Moreover, since the hooking part 132 is attachable to and detachable from the cable part 104, the hooking part 132 is detached from the cable part 104, so that the hooking part 132 does not become obstructive in a case where the endoscope 100 is used alone, and cleaning and sterilization of mounting portions (the smaller-diameter part 152 of the cable part 104, the locking hole 146 of and the hooking part 132, and the like) of the hooking part 132 can be easily performed.

Subsequently, the action of the forward and backward movement operating part 130 of an endoscope 100 will be described.

As illustrated in Fig. 1, a reduction in the diameter of the overtube is achieved, and the forward and backward movement operating part 130 of the endoscope 100 and the operating part 204 of the treatment tool 200 are arranged in proximity to each other by the diameter of the overtube 300 being reduced, in a state where the endoscope 100 and the treatment tool 200 are respectively inserted through the endoscope insertion passage 306 and the treatment tool insertion passage 308 of the overtube 300, and the position of the distal end surface 114 of the endoscope 100 in the axial direction (forward-backward direction) is adjusted with respect to the position of the treatment part 206 of the treatment tool 200 so that the treatment part 206 is reflected on an endoscope image.

In this case, a portion of the operating part 204 of the treatment tool 200 may overhang the axis of the endoscope 100 depending on the size of the operating part 204 of the treatment tool 200. However, even in that case, the forward and backward movement operating part 130 does not hinder that the cable part 104 of the endoscope 100 is bent. Thus, when the cable part 104 is bent, it is possible to insert and arrange the endoscope 100 and the treatment tool 200 through the endoscope insertion passage 306 and the treatment tool insertion passage 308 of the overtube 300, and the forward and backward movement operating part 130 of the bent cable part 104 and the curved operating part 204 of the treatment tool 200 can be arranged to approach or contact each other.

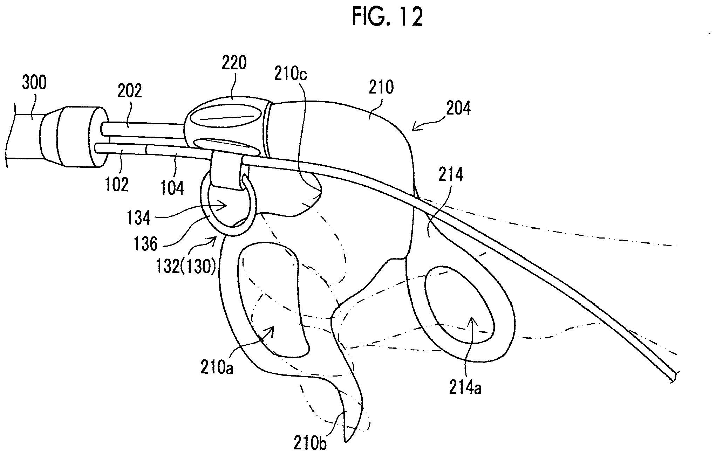

Meanwhile, as illustrated in Fig. 12, a surgeon usually grips the operating part 204 of the treatment tool 200 with his/her right hand to perform a required operation, the middle finger and the third finger of his/her right hand are inserted into a fixed ring part 210a of the fixed handle 210, and the thumb is inserted into a movable ring part 214a of the movable handle 214. The little finger is hooked on a circular arc part 210b provided to extend to a lower side of the fixed ring part 210a, and the index finger is hooked on a circular arc part 210c provided on an upper side of the fixed ring part 210a.

In a case where the gripping members of the treatment part 206 are opened and closed and in a case where the surgeon's thumb is moved forward and backward to move the movable handle 214 and rotate the gripping members of the treatment part 206, the index finger is released from the circular arc part 210c and is made to abut against the side surface of the rotating handle 220 forward, and the index finger is moved in a desired rotational direction to rotate the rotating handle 220.

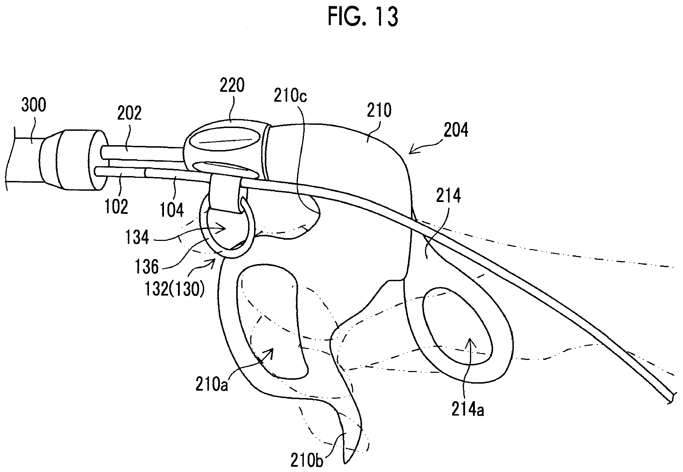

In addition, in Fig. 12, for convenience, only one hooking part 132 in the forward and backward movement operating part 130 of the endoscope 100 is illustrated, and the other two are omitted (the same applies to Fig. 13).

If the cable part 104 (forward and backward movement operating part 130) of the endoscope 100 is arranged on the left side with respect to the operating part 204 of the treatment tool 200 in a state the operating part 204 of the treatment tool 200 is gripped with the right hand in this way, and the index finger of the right hand is extended toward the left side, the index finger can be passed through the opening 134 of any hooking part 132 of the three hooking parts 132 of the forward and backward movement operating part 130 of the endoscope 100 as illustrated in Fig. 13. Accordingly, the endoscope 100 and the index finger can be engaged with each other, and a state where the forward and backward movement operation of the endoscope 100 is possible only with the right hand without releasing the right hand gripping the treatment tool 200 from the treatment tool 200 is brought about.

Then, if the index finger passed through the opening 134 is moved backward to move the ring member 136 backward inside the index finger, only the endoscope 100 can be moved backward with respect to the treatment tool 200 in an interlocking manner with this.

On the other hand, if the index finger passed through the opening 134 is moved forward to move the ring member 136 forward outside the index finger, only the endoscope 100 can be moved forward with respect to the treatment tool 200 in an interlocking manner with this.

Accordingly, it is possible to perform change to a visual field suitable for detailed observation and treatment, and surgical efficiency can be improved.

Additionally, if the overall right hand is moved in the forward-backward direction without moving the index finger passed through the opening 134 to move the treatment tool 200 forward and backward in the forward-backward direction, it is also possible to forcibly invalidate the action of the dead zone of the slider 400 of the overtube 300 to move the endoscope 100 forward and backward together with the treatment tool 200.

In addition, by bending a first joint of the index finger passed through the opening 134 to pull the ring member 136 closer to the operating part 204 side of the treatment tool 200, the cable part 104 can be constrained to the operating part 204 side of the treatment tool 200, and the endoscope 100 can be operated to move forward and backward in a stable state, without also causing a situation in which the cable part 104 is bent in a direction away from the operating part 204 of the treatment tool 200 and the index finger slips out of the opening 134, during the operation as above.

Additionally, while the index finger of the right hand is used for the operation of the forward and backward movement operating part 130 of the endoscope 100, the index finger cannot be used for the operation of the treatment tool 200. However, since there is little frequency in use of the index finger and the index finger most easily accesses the forward and backward movement operating part 130, the forward and backward movement operating part 130 is operated by the index finger of the right hand.

Here, the installation position of the hooking part 132 will be described.

A case where the endoscope 100 is used after being pulled to the rearmost side with respect to the treatment tool 200 in a state where the endoscope 100 and the treatment tool 200 are respectively inserted through the endoscope insertion passage 306 and the treatment tool insertion passage 308 of the overtube 300 as illustrated in Fig. 1 is assumed. That is, it is supposed that there is no state where the endoscope 100 is further pulled backward with respect to the treatment tool 200 more than this or the endoscope 100 is further pushed forward with respect the treatment tool 200.

In this case, if the length from the distal end (the distal end of each gripping member) of the treatment part 206 of the treatment tool 200 to the circular arc part 210c on which the index finger of the operating part 204 is hooked is L1, and the distance in the forward-backward direction from the distal end of the treatment part 206 to the distal end surface 114 of the endoscope 100 is L2, it is desirable to adopt a configuration in which the condition that the length from the distal end surface 114 of the endoscope insertion part 102 to the base end thereof is shorter than at least a length (L1 - L2), that is, the condition that the length is shorter than the distance in the forward-backward direction from the position of the distal end surface 114 of the endoscope 100 to the circular arc part 210c is satisfied. Accordingly, the cable part 104 of the endoscope 100 can always be arranged at a peripheral part of the operating part 204 (circular arc part 210c) of the treatment tool 200, and any interference between the operating part 204 of the treatment tool 200 and the endoscope 100 is prevented.

As a more preferable form, the forward and backward movement operating part 130 is provided so that the position of the cable part 104 with the length (L1 - L2) from the distal end surface 114 of the endoscope 100 becomes a distal end position within a range of the forward and backward movement operating part 130. That is, the smaller-diameter part 152 on the foremost side of the three smaller-diameter parts 152 on which the hooking parts 132 are mounted is formed at a position with the length (L1 - L2) from the distal end surface 114 of the endoscope 100.

Accordingly, the hooking part 132 on the foremost side of the three hooking parts 132 of the forward and backward movement operating part 130 is arranged at an optimum use position (a position where the index finger is easily passed therethrough) in a state where the endoscope 100 is used after being pulled to the rearmost side with respect to the treatment tool 200.

Additionally, if the distance in the forward-backward direction from the distal end of the treatment part 206 to the distal end surface 114 of the endoscope 100 is L3 in a case where it is assumed that the endoscope 100 is used after being pushed to the foremost side with respect to the treatment tool 200, it is preferable to provide the forward and backward movement operating part 1 30 so that the position of the cable part 104 with the length (L1 - L3) from the distal end surface 114 of the endoscope 100 becomes a rear end position within the range of the forward and backward movement operating part 130. That is, the smaller-diameter part 152 on the rearmost side of the three smaller-diameter parts 152 on which the hooking parts 132 are mounted is formed at a position with a length (L1 - L3) from the distal end surface 114 of the endoscope 100.

Accordingly, the hooking part 132 on the rearmost side of the three hooking parts 132 of the forward and backward movement operating part 130 is arranged at an optimum use position in a state where the endoscope 100 is used after being pulled to the foremost side with respect to the treatment tool 200.

Additionally, it is preferable to form the central smaller-diameter part 152 of the three smaller-diameter parts 152 on which the hooking parts 132 are mounted, at a central position between the smaller-diameter part 152 on the foremost side and the smaller-diameter part 152 on the rearmost side, and arrange the three hooking parts 132 at the positions with equal intervals along the cable part 104.

From the above, even if the three smaller-diameter parts 152 are formed at equal intervals in an axial range of the cable part 104 of the endoscope 100 that may be arranged at a position adjacent to the operating part 204 of the treatment tool 200, that is, in a range where the length from the distal end surface 114 of the endoscope 100 is equal to or more than (L1 - L2) and equal to or less than (L1 - L3), and the positional relationship between the treatment tool 200 and the endoscope 100 varies, any hooking part 132 of the three hooking parts 132 is arranged at a position suitable for use. Here, L1 > L2 > L3 is established.

In addition, in a case where the forward and backward movement operating part 130 is arranged on the above conditions and the intervals of the hooking part s132 are too large, four or more smaller-diameter parts 152 may be formed in the cable part 104 so as to have suitable intervals, and hooking parts 132 equivalent to the number of smaller-diameter parts may be made mountable.

Additionally, the invention is limited to the above conditions, and an arbitrary number of smaller-diameter parts 152 may be formed at regular intervals over a wide range of the cable part 104, and the hooking parts 132 may be mounted at required positions, or the number of hooking parts 132 to be mounted on the cable part 104 may be only one or may be two or more. Additionally the intervals of the smaller-diameter parts 152 are not necessarily constant, either.

Moreover, the configuration of the coupling member 138 that detachably mounts the ring member 136 having the opening 134 on the cable part 104 is not limited to the above-described form. It is also possible to adopt a configuration in which the ring member 136 (a member having the opening 134) is detachably mounted on an arbitrary position of the cable part 104 without providing the smaller-diameter parts 152 in the cable part 104. In that case, an arbitrary number of openings 134 can also be arranged at arbitrary intervals in the cable part 104.

Next, an example of the forward and backward movement operation of the endoscope 100 and the treatment tool 200 in the endoscopic surgical device 10 of the present embodiment will be described.

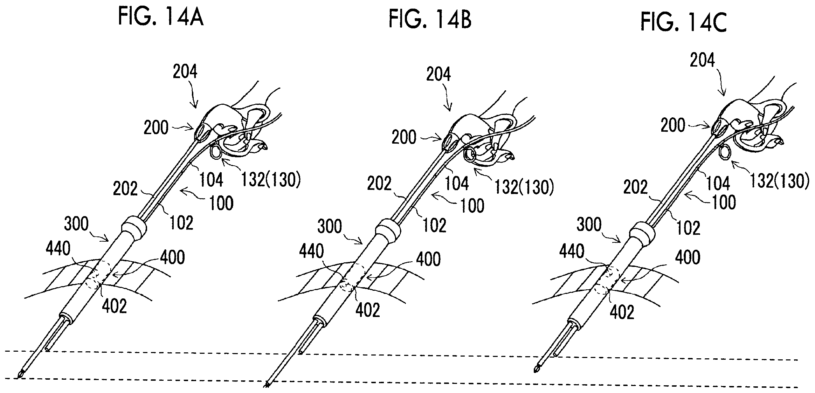

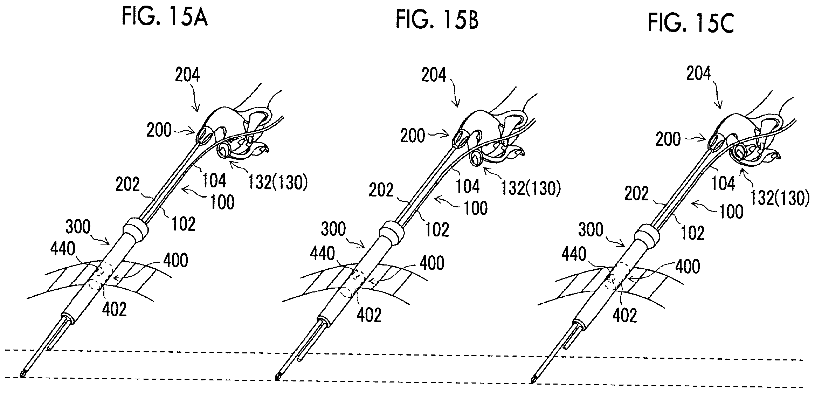

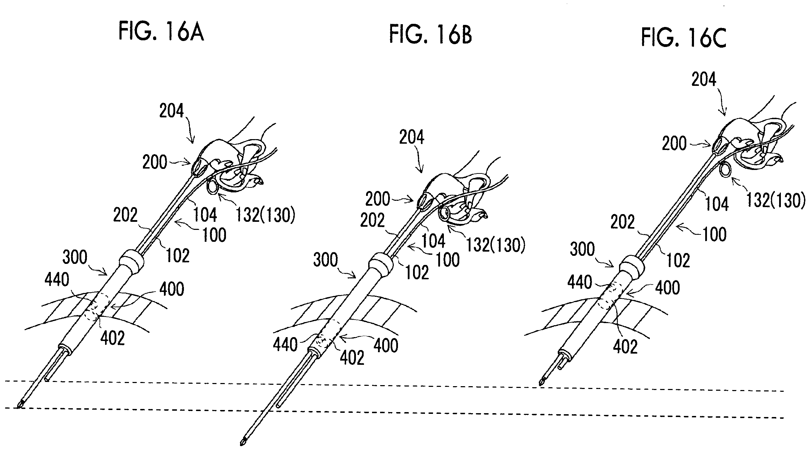

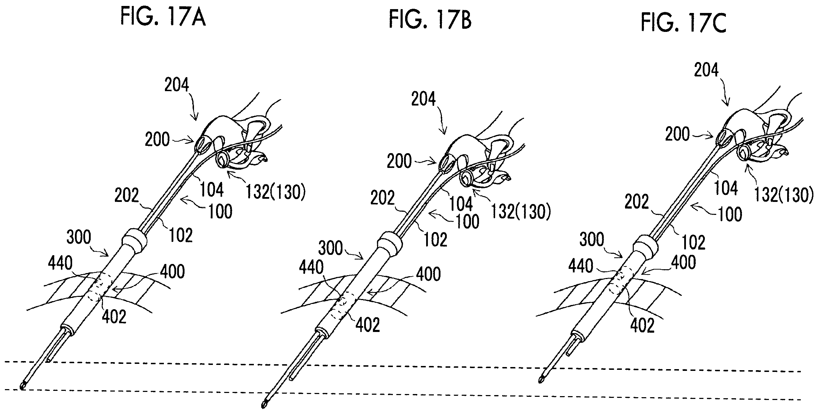

Figs. 14A to 17C are explanatory views illustrating the aspect of the operation when treatment of a diseased site within a patient's body cavity is performed using the endoscopic surgical device 10 of the present embodiment, Figs. 14A to 14C illustrate an aspect of the operation when only the treatment tool 200 moves forward and backward, Figs. 15A to 15C illustrate an aspect of the operation when only the endoscope 100 moves forward and backward, Figs. 16A to 16C illustrate an aspect of the operation when the endoscope 100 moves forward and backward in an interlocking manner with the treatment tool 200 through an interlocking function of the slider 400, and Figs. 17A to 17C illustrate an aspect of the operation when the endoscope 100 moves forward and backward in an interlocking manner with the treatment tool 200 through the interlocking function of the slider 400. In addition, in these drawings., one hooking part 132 in the forward and backward movement operating part 130 of the endoscope 100 is illustrated.

As illustrated in Fig. 14A, the endoscope 100 (endoscope insertion part 102) and the treatment tool 200 (treatment tool insertion part 202) are respectively inserted into the endoscope insertion passage 306 and the treatment tool insertion passage 308 of the overtube 300 after the overtube 300 is inserted into a patient's body wall and a pneumoperitoneum gas is injected into a body cavity. In this case, the endoscope 100 is coupled to the slider body 402 of the slider 400, and the treatment tool 200 is coupled to the sleeve 440 of the slider 400. Thus, when the sleeve 440 moves within a movable range thereof with respect to the slider body 402, the interlocking is performed with the dead zone (play) where the endoscope 100 does not interlock with the forward and backward movement of the treatment tool 200.

In this state, if the surgeon grips only the operating part 204 of the treatment tool 200 and minutely moves only the treatment tool 200 forward, only the treatment tool 200 can be moved forward in a state where the endoscope 100 is made stationary as illustrated in Fig. 14B, with respect to the forward movement in the dead zone until the sleeve 440 of the slider 400 abuts against the front end of the movable range thereof.

Similarly, if the surgeon grips only the operating part 204 of the treatment tool 200 and minutely moves only the treatment tool 200 backward, only the treatment tool 200 can be moved backward in a state where the endoscope 100 is made stationary as illustrated in Fig. 14C, with respect to the backward movement in the dead zone until the sleeve 440 of the slider 400 abuts against the rear end of the movable range thereof.

Therefore, since the endoscope 100 does not move forward and backward with respect to the minute forward and backward movement operation of the treatment tool 200, the range of an observation image displayed on the monitor 112 does not change, the size of an object to be observed can be prevented from fluctuating according to the minute displacement of the treatment tool 200, a sense of perspective can be suitably maintained, and a stable observation image can be obtained.

Fig. 15A illustrates that the overtube 300, the endoscope 100, and the treatment tool 200 are in the same state as those of Fig. 14A.

In this state, if the surgeon passes the index finger of his/her right hand gripping the operating part 204 of the treatment tool 200 through the opening 134 and moves the index finger forward, only the endoscope 100 can be moved forward in a state where the treatment tool 200 is made stationary as illustrated in Fig. 15B, with respect to the forward movement in the dead zone until the sleeve 440 of the slider 400 abuts against the rear end of the movable range thereof.

Similarly, if the surgeon passes the index finger of his/her right hand gripping the operating part 204 of the treatment tool 200 through the opening 134 and moves the index finger backward, only the endoscope 100 can be moved backward in a state where the treatment tool 200 is made stationary as illustrated in Fig. 15C, with respect to the backward movement in the dead zone until the sleeve 440 of the slider 400 abuts against the front end of the movable range thereof.

Therefore, since the position of the distal end surface 114 of the endoscope 100 can be moved forward and backward without the treatment tool 200 moving forward and backward with respect to the forward and backward movement operation of the endoscopes 100, only the visual field (observation range) of the endoscope 100 can be changed. That is, the observation range can be magnified or diminished (the size of an object to be observed in the observation image is increased or reduced).

Fig. 16A illustrates that the overtube 300, the endoscope 100, and the treatment tool 200 are in the same state as those of Fig. 14A.

In this state, if the surgeon grips only the operating part 204 of the treatment tool 200 and greatly moves the treatment tool 200 forward, the endoscope 100 can be moved forward in an interlocking manner with the forward movement of the treatment tool 200 through the interlocking function of the slider 400 as illustrated in Fig. 16B, after the forward movement in the dead zone until the sleeve 440 of the slider 400 abuts against the front end of the movable range thereof.

Similarly, if the surgeon grips only the operating part 204 of the treatment tool 200 and greatly moves the treatment tool 200 backward, the endoscope 100 can be moved backward in an interlocking manner with the backward movement of the treatment tool 200 through the interlocking function of the slider 400 as illustrated in Fig. 16C, after the backward movement in the dead zone until the sleeve 440 of the slider 400 abuts against the rear end of the movable range thereof.

Therefore, since the endoscope 100 moves forward and backward with respect to a large forward and backward movement operation of the treatment tool 200, the range of an observation image displayed on the monitor 112 is continuously changed so as to follow the forward and backward movement of the treatment tool 200. Accordingly, since the size of an object to be observed changes according to the operation of the treatment tool 200, an image desired by a surgeon can be simply obtained.

Fig. 17A illustrates that the overtube 300, the endoscope 100, and the treatment tool 200 are in the same state as those of Fig. 14A.

In this state, if the surgeon passes the index finger of his/her right hand gripping the operating part 204 of the treatment tool 200 through the opening 134 and moves the whole right hand forward with the index finger fixed, the endoscope 100 can be moved forward together with the treatment tool 200 as illustrated in Fig. 17B even in a state where the sleeve 440 of the slider 400 does not abuts against the front end of the movable range thereof.

Similarly, if the surgeon passes the index finger of his/her right hand gripping the operating part 204 of the treatment tool 200 through the opening 134 and moves the whole right hand backward with the index finger fixed, the endoscope 100 can be moved backward together with the treatment tool 200 as illustrated in Fig. 17C even in a state where the sleeve 440 of the slider 400 does not abuts against the rear end of the movable range thereof.

Therefore, the dead zone of the slider 400 with which the endoscope 100 does not interlock can be invalidated with respect to the forward and backward movement operation of the treatment tools 200 and the endoscope 100. Thus, the endoscope 100 can be moved forward and backward more immediately than the forward and backward movement of the endoscope 100 through the interlocking function of the slider 400 described in Figs. 16A to 16C. In a case where the surgeon tries to immediately move the endoscope 100 forward and backward together with the treatment tool 200, the dead zone can be invalidated by selecting such an operation.

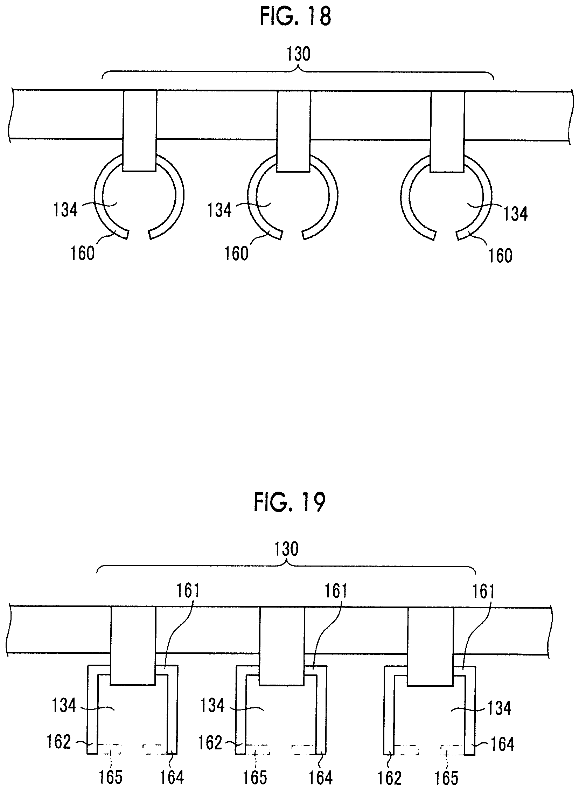

As described above, in the forward and backward movement operating part 130 of the endoscope 100 of the above embodiment, the opening 134 which a finger is passed through and hooked on is provided by the ring-shaped ring member 136. However, the opening 134 is not necessarily ring-shaped. For example, forms as illustrated in Figs. 18 and 19 may be provided.

In Figs. 18 and 19, if members that are the same as or similar to as the form of Figs. 10 and 11 are designated by the same reference signs and the description thereof is omitted, as illustrated in Fig. 18, a form in which the opening 134 through which the index finger is passed is formed by a C-shaped member 160 obtained by cutting out a portion of an endless ring member may be adopted.

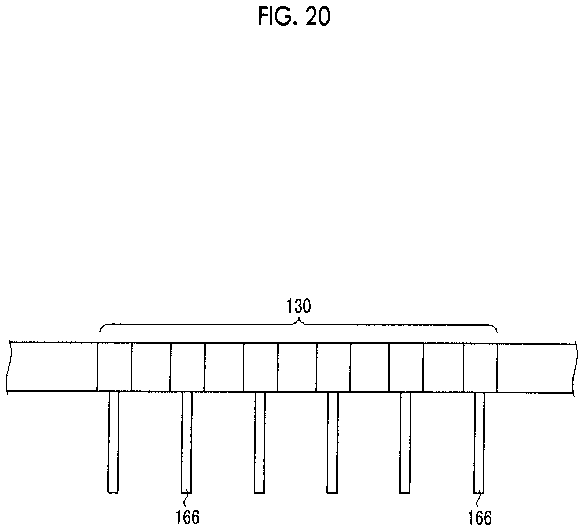

Additionally, as illustrated in Fig. 19, a form may be adopted in which two rod-shaped members 162 and 164 extending in an orthogonal direction from the both ends of a linear rod-shaped member 161 that run in the direction of the central axis of the cable part 104 and is held by the coupling member 138 are provided, and a space between the two rod-shaped members 162 and 164 is used as the opening 134 through which the index finger is passed. In addition, if projections (projections 165 illustrated by dashed lines) that protrude in an orthogonal direction from distal ends of the rod-shaped members 162 and 164 are provided, a finger passed through the opening 134 can be prevented from slipping out unintentionally. Moreover, as illustrated in Fig. 20, a plurality of rod-shaped members 166 extending in a direction orthogonal to a central axis of the cable part 104 may be provided at regular intervals, and a space between two arbitrary rod-shaped members 166 adjacent to each other may be used as the opening 134 through which the index finger is passed.

Additionally, the overtube 300 of the above embodiment includes an interlocking mechanism that interlocks the endoscope 100 (endoscope insertion part 102) and the treatment tool 200 (treatment tool insertion part 202), which are respectively inserted through the endoscope insertion passage 306 and the treatment tool insertion passage 308 of the overtube 300, with each other with play using the slider 400. However, the invention is effective also in a case where an overtube, which includes an endoscope insertion passage through which the endoscope 100 (endoscope insertion part 102) is inserted and a treatment tool insertion passage through which the treatment tool 200 (treatment tool insertion part 202) is inserted, does not include an interlocking mechanism having play unlike the slider 400, and allows the endoscope 100 and the treatment tool 200 to move forward and backward in an interlocking manner with each other, is used. That is, only the endoscope 100 can be moved forward and backward only with the right hand, which grips the operating part 204 of the treatment tool 200, by the operation as illustrated in Figs. 15A to 15C, and the endoscope 100 can be moved forward and backward together with the treatment tool 200 only with the right hand, which grips the operating part 204 of the treatment tool 200, by the operation as illustrated in Figs. 17A to 17C.

Additionally, a case where the operating part 204 of the treatment tool 200 is gripped with the right hand and the forward and backward movement operating part 130 of the endoscope 100 is operated with the index finger of the right hand has been described in the above embodiment. However, depending on the state of the right hand that grips the operating part 204 of the treatment tool 200, the forward and backward movement operating part 130 of the endoscope 100 may be operated with fingers other than the index finger. Additionally, in a case where the operating part 204 of the treatment tool 200 is gripped with the left hand, it is possible to arrange the endoscope 100 on the right side of the treatment tool 200, thereby operating the forward and backward movement operating part 130 of the endoscope 100 with a finger with the left hand.

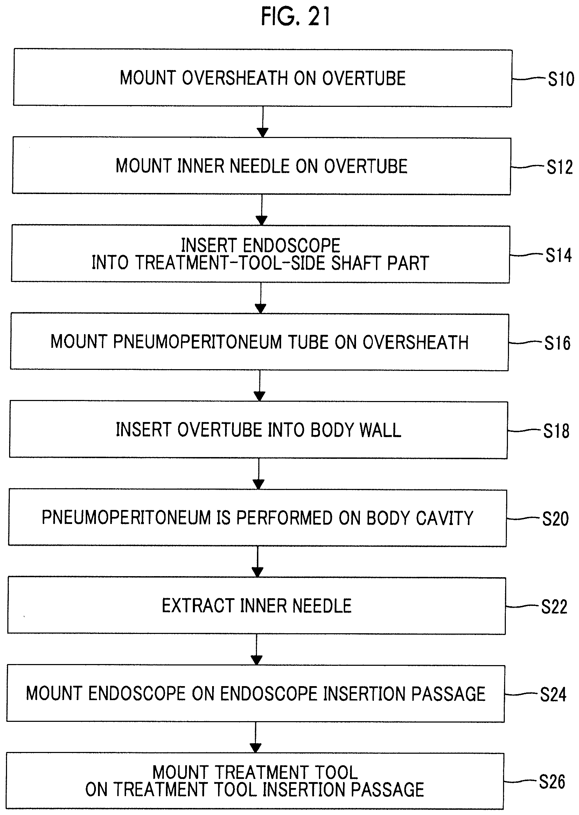

Next, as illustrated in Fig. 14A, a procedure when inserting the overtube 300 into a patient's body wall will be described according to a flowchart of Fig. 21.

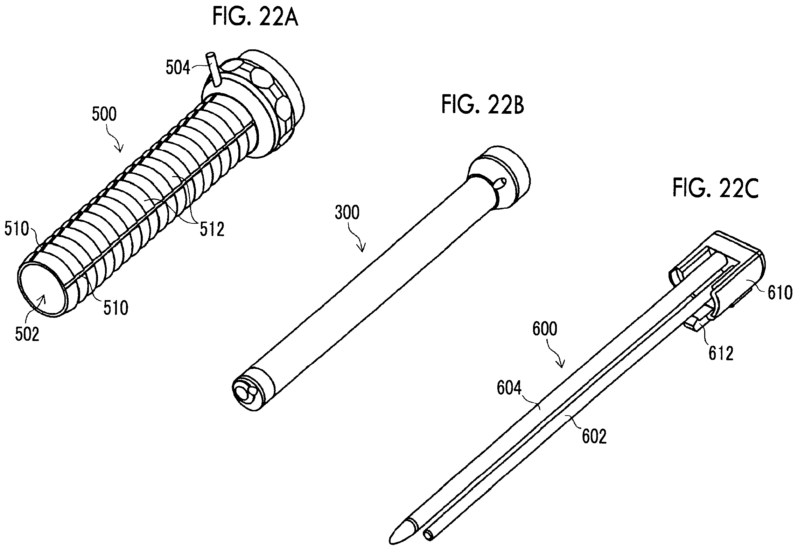

Here, as illustrated in Figs. 22A to 22C, a case where the oversheath 500 fitted to the overtube 300 and the inner needle 600 inserted into the endoscope insertion passage 306 and the treatment tool insertion passage 308 of the overtube 300 are used in addition to the above-described overtube 300 is used is described. However, the configuration and action of the oversheath 500 and the inner needle 600 will be appropriately described below.

First, as a process of Step S10 of Fig. 21, the oversheath 500 is mounted on the overtube 300 before the overtube 300 is inserted into a patient's body wall.

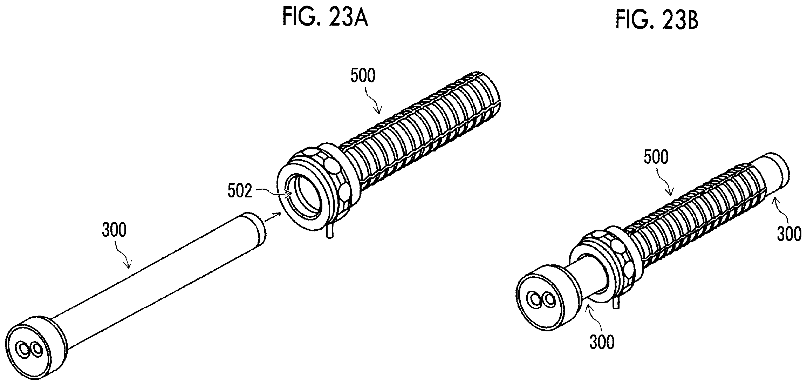

As illustrated in Fig. 22A, the oversheath 500 is formed in a long tubular shape, and has an insertion hole 502 passing therethrough from a base end to a distal end. By inserting the overtube 300 into the insertion hole 502 of the oversheath 500 from a distal end side as illustrated in Fig. 23A, the oversheath 500 is fitted to and mounted on an outer peripheral part of the overtube 300 as illustrated in Fig. 23B.

As illustrated in Fig. 22A, irregularities consisting of, for example, four longitudinal grooves 510 that restrict the rotation of the overtube in a direction around an axis with respect to the body wall, and a number of lateral groove 512 that restricts the forward and backward movement of the overtube in the axial direction with respect to the body wall are formed in an outer peripheral part of the oversheath 500. Thus, when the oversheath 500 is mounted on the overtube 300, the overtube 300 is restrained from unintentionally fluctuating with respect to the body wall (the rotation of the overtube around the axis and the forward and backward movement of the overtube in the axial direction) after the overtube 300 is inserted into the body wall.

Additionally, an airtight elastic member (valve member or the like) is arranged on a base end side of the insertion hole 502 of the oversheath 500. Thus, if the overtube 300 is inserted through the insertion hole 502 of the oversheath 500 like Fig. 23B, airtightness is made on the base end side between an inner peripheral surface of the oversheath 300 and an outer peripheral surface of the overtube 300 by the airtight elastic member.

First, as a process of Step S12 of Fig. 21, the inner needle 600 is mounted on the overtube 300 (refer to Fig. 22B).

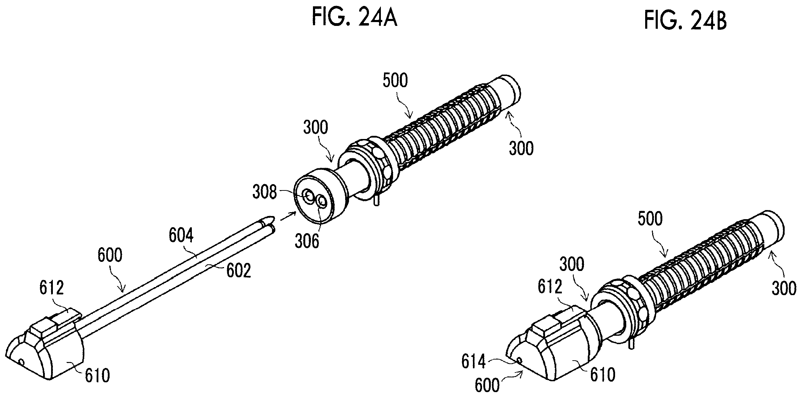

As illustrated in of Fig. 22C, the inner needle 600 has an elongated endoscope-side shaft part 602 and an elongated treatment-tool-side shaft part 604 that are provided to extend from a head part 610 on the base end side toward the distal end side. By respectively inserting the endoscope-side shaft part 602 and the treatment-tool-side shaft part 604 into the endoscope insertion passage 306 and the treatment tool insertion passage 308 of the overtube 300 as illustrated in Fig. 24A and by engaging an engaging claw of a locking lever 612 provided in the head part 610 with a locking hole (not illustrated) of an outer peripheral part (an outer peripheral part of the base end cap 340) on the base end side of the overtube 300, the inner needle 600 is mounted on the overtube 300 as illustrated in Fig. 24B. In this case, airtightness is made on the base end side between an outer peripheral surface of the endoscope-side shaft part 602 and an inner peripheral surfaces of the endoscope insertion passage 306 and between an outer peripheral surface of the treatment-tool-side shaft part 604 and an inner peripheral surfaces of the treatment tool insertion passage 308 by the valve members 346 and 348 (refer to Fig. 4) provided on respective base end sides of the endoscope insertion passage 306 and the treatment tool insertion passage 308 of the overtube 300.

Additionally, if the inner needle 600 is mounted on the overtube 300, a distal end of the treatment-tool-side shaft part 604 of the inner needle 600 inserted through the treatment tool insertion passage 308 of the overtube 300 is arranged at a position where the distal end protrudes from a distal end of the overtube 300. In addition, a distal end of the endoscope-side shaft part 602 of the inner needle 600 inserted into the endoscope insertion passage 306 of the overtube 300 is arranged on the same surface as the distal end or at a position where the distal end protrudes further than the distal end of the overtube 300.

Next, as a process of Step S14 of Fig. 21, the endoscope 100 (endoscope insertion part 102) is inserted into a hole of the treatment-tool-side shaft part 604 of the inner needle 600.

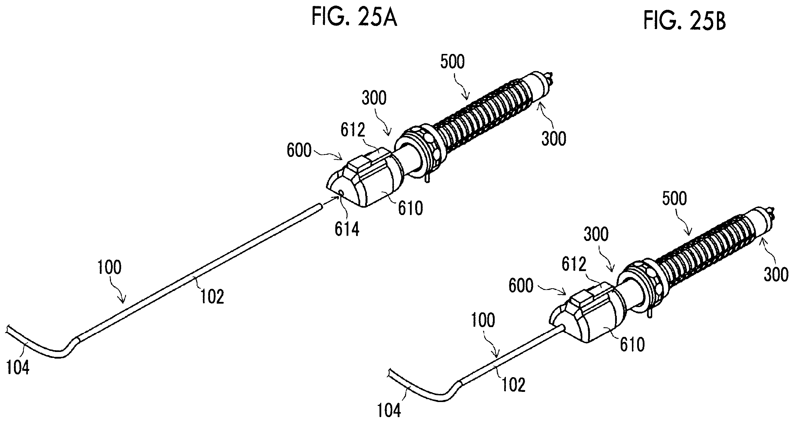

The treatment-tool-side shaft part 604 of the inner needle 600 has a larger external diameter than that of the endoscope-side shaft part 602, and has a hole with a larger internal diameter than that of the endoscope insertion part 102 therein. As illustrated in Fig. 25A, a base end surface of the head part 610 of the inner needle 600 is provided an opening 614 that communicates with the hole inside the treatment-tool-side shaft part 604. By inserting the endoscope 100 (endoscope insertion part 102) from the opening 614 and striking the distal end of the endoscope insertion part 102 against a distal end inside the hole of the treatment-tool-side shaft part 604, the endoscope 100 is mounted on the treatment-tool-side shaft part 604 as illustrated in Fig. 25B.

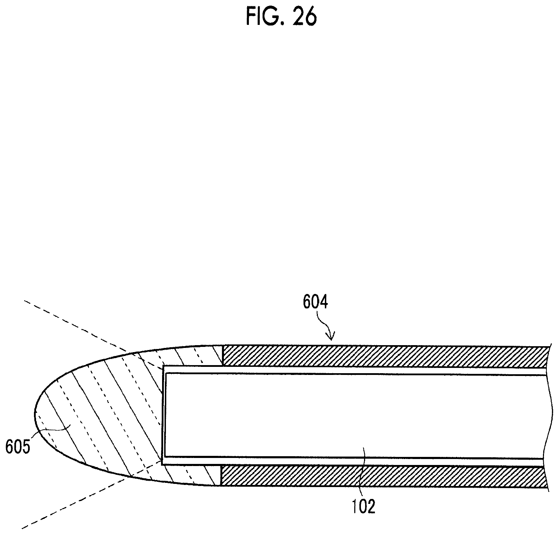

As illustrated in Fig. 26, an optically transparent cap member 605 is attached to the distal end of the treatment-tool-side shaft part 604 of the inner needle 600, and the hole inside the treatment-tool-side shaft part 604 is formed to communicate with the inside of the cap member 605. If the endoscope 100 is inserted into the hole of the treatment-tool-side shaft part 604, the distal end of the endoscope insertion part 102 is arranged at a position where the distal end thereof strikes the distal end of the hole of the cap member 605. Accordingly, a peripheral part of the distal end of the overtube 300 is imaged via the cap member 605 by the observation part provided at the distal end of the endoscope insertion part 102, so that the resulting image can be observed the monitor 112.

Additionally, although the distal end of the cap member 605 of the treatment-tool-side shaft part 604 is formed in a rounded non-edge shape so that an edge is not made in a curved surface shape, this distal end is formed in a tapered shape so that the distal end can pass through the body wall easily.

In addition, the treatment-tool-side shaft part 604 may be formed of the optically transparent member not only at the distal end but also in its entirety. Additionally, in the overtube 300 of the present embodiment, the diameter of the endoscope insertion part 102 inserted through the endoscope insertion passage 306 is about 3.0 mm, and the diameter of the treatment tool insertion part 202 inserted through the treatment tool insertion passage 308 is about 5.0 mm that becomes mainstream in surgical treatment. In this case, the endoscope-side shaft part 602 of the inner needle 600 and the treatment-tool-side shaft part 604 are formed so that the diameters thereof become approximately equal to the diameter of the endoscope insertion part 102 and the treatment tool insertion parts 202, and have a diameter of about 3.0 mm and a diameter of about 5.0 mm, respectively. Therefore, by providing a hole in the treatment-tool-side shaft part 604 such that the diameter of the treatment-tool-side shaft part 604 of the inner needle 600 is larger than the diameter of the endoscope insertion part 102, the endoscope insertion part 102 is insertable into the hole.

Next, as a process of Step S16 of Fig. 21, a pneumoperitoneum tube 700 is mounted on the oversheath 500.

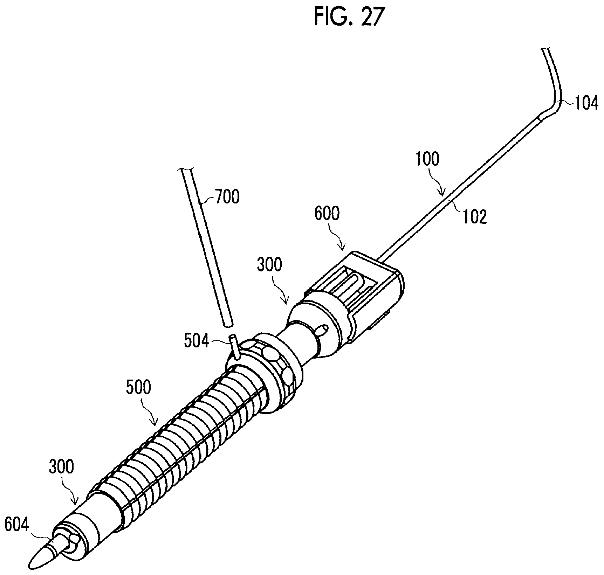

As illustrated in Figs. 22A to 22C, a head part of the oversheath 500 is provided with a tubular tube connector 504, and as illustrated in Fig. 27, the pneumoperitoneum tube 700 is mounted on the oversheath 500 by connecting one end of the pneumoperitoneum tube 700 to the tube connector 504.

The other end of the pneumoperitoneum tube 700 is connected to a pneumoperitoneum device (not illustrated), and a pneumoperitoneum gas, such as carbon dioxide, is set to a state where this gas is capable of being supplied from the pneumoperitoneum device through a pipe line of the pneumoperitoneum tube 700 to the oversheath 500.

In addition, illustration of the pneumoperitoneum tube 700 is omitted in the drawings to be used below.

Next, as a process of Step S18 of Fig. 21, the body wall (for example, an abdominal wall) is punctured by the overtube 300.

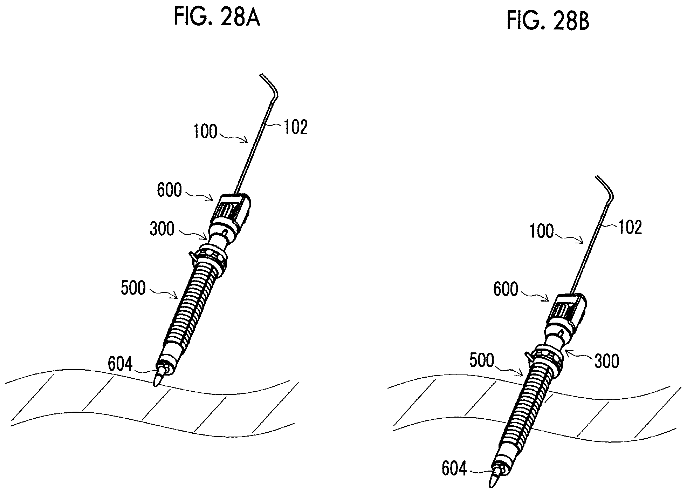

That is, an incision is made in a skin portion of the body wall into which the overtube 300 is inserted, and as illustrated in Fig. 28A, the distal end of the treatment-tool-side shaft part 604 of the inner needle 600 protruding from the distal end of the overtube 300 is inserted into an incised portion. The overtube 300 is bluntly inserted. Accordingly, as illustrated in Fig. 28B, the irregularities of the outer peripheral part of the oversheath 500 come into contact the body wall, and the distal end of the overtube 300 is arranged at a position within the body cavity so that the overtube 300 is inserted through the inside of the insertion hole 502 of the oversheath 500. In this case, by imaging the distal end peripheral part of the overtube 300 with the endoscope 100 mounted on the treatment-tool-side shaft part 604 of the inner needle 600 and viewing the resulting image, it can be checked that the overtube 300 has reached the inside of the body cavity and is not puncturing other internal organs.

Next, as a process of Step S20 of Fig. 21, pneumoperitoneum is performed on the body cavity (for example, an abdominal cavity).

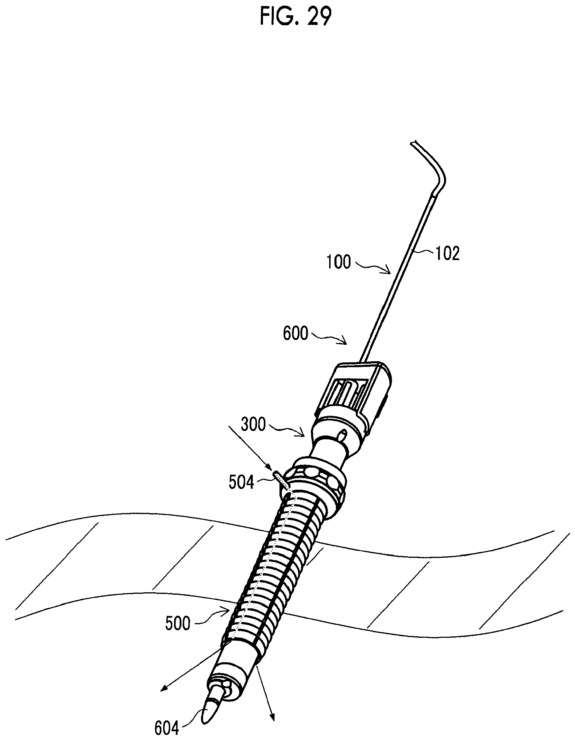

That is, the pneumoperitoneum tube 700 is connected to the tube connector 504 of the oversheath 500 in the process of Step S16, and a pneumoperitoneum gas is supplied from the pneumoperitoneum device through the pneumoperitoneum tube 700 to the tube connector 504 of the oversheath 500. The tube connector 504 of the oversheath 500 has a pipe line that communicates with the insertion hole 502 of the oversheath 500, and as illustrated in Fig. 29, the pneumoperitoneum gas supplied to the tube connector 504 through the pneumoperitoneum tube 700 is supplied into the body cavity from the distal end of the oversheath 500 through a gap between an inner peripheral surface of the insertion hole 502 of the oversheath 500 and the outer peripheral surface of the overtube 300. Accordingly, the pneumoperitoneum is performed.

Next, as a process of Step S22 of Fig. 21, the inner needle 600 is extracted from the overtube 300.

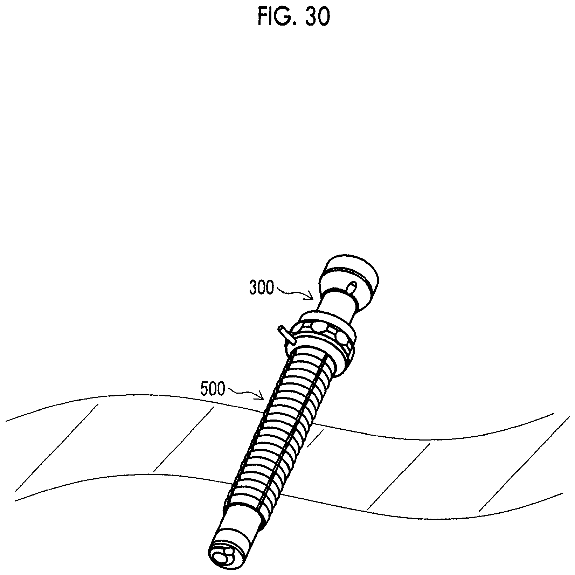

Accordingly, as illustrated in Fig. 30, the overtube 300 and the oversheath 500 remain in the body wall.

Next, as a process of Step S24 of Fig. 21, the endoscope 100 (endoscope insertion part 102) is mounted on the endoscope insertion passage 306 of the overtube 300.

Subsequently, as a process of Step S26 of Fig. 21, the treatment tool 200 (treatment tool insertion part 202) is mounted on the treatment tool insertion passage 308 of the overtube 300.

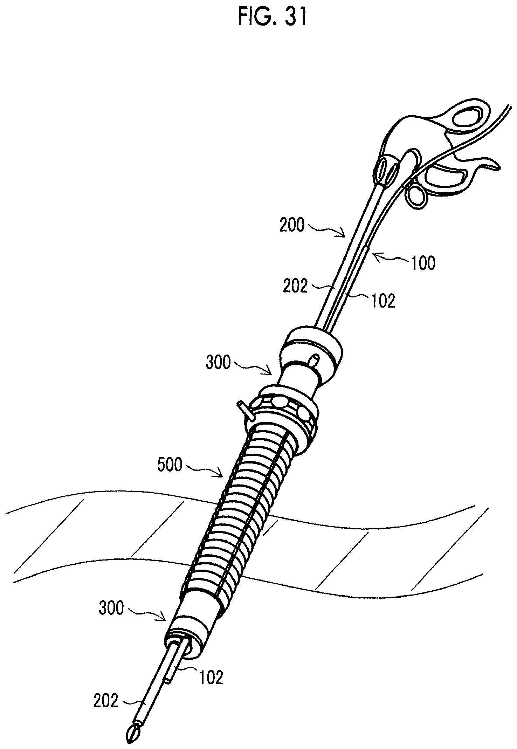

Accordingly, as illustrated in Fig. 31, the distal end of the endoscope insertion part 102 and the distal end of the treatment tool insertion part 202 are arranged within the body cavity via the overtube 300 in the body wall.

According to the above procedure, when the overtube 300 inserted into a body wall, the periphery of the distal end of the overtube 300 can be visually checked with the endoscope 100. Therefore, a body wall breakthrough is easily checked, and puncturing into other internal organs can be reliably prevented. Therefore, procedure efficiency can be enhanced.

Additionally, by adopting a configuration in which the endoscope 100 is arranged in the hole of the treatment-tool-side shaft part 604 with an external diameter greater than the endoscope 100, it is not necessary to provide a portion for mounting the endoscope 100 on the overtube 300 for the checking of the body wall breakthrough. That is, since it is not necessary to enlarge the diameter of the overtube 300, there is an effect that an invasion to the body wall is reduced.

### Explanation of References

10: endoscopic surgical device
100: endoscope
102: endoscope insertion part
104: cable part
108: processor device
110: light source device
112: monitor
114, 304: distal end surface
116: observation window
118: illumination window
130: forward and backward movement operating part
132: hooking part
134: opening
136: ring member
138: coupling member
140: holding part
142: locking part
142a: plate-shaped body
150: larger-diameter part
152: smaller-diameter part
160: thin C-shaped member
161, 162, 164, 166: rod-shaped member
165: projection
200, 206: treatment tool
202: treatment tool insertion part
204: operating part
206: treatment part
208: sheath
210: fixed handle
210a: fixed ring part
210b, 210c: circular arc part
214: movable handle
214a: movable ring part
220: rotating handle
300: overtube
300a: reference axis
302: base end surface
306: endoscope insertion passage
306a: endoscope insertion axis
308: treatment tool insertion passage
310: endoscope insertion port
312: endoscope delivery port
314: treatment tool insertion port
316: treatment tool delivery port
320: overtube body
340: base end cap
360: distal end cap
400: slider
402: slider body
420: endoscope-coupled part
422: treatment tool-coupled part
426, 446: pressure-contact member
440: sleeve
500: oversheath
600: inner needle

## Claims

1. An endoscope (100) adapted to be used for an endoscopic surgical device (10) including the endoscope (100) wherein the endoscope comprises an endoscope insertion part (102) having an observation part (116) provided at a distal end thereof and a flexible cable (104) connected to a base end of the endoscope insertion part (102), and wherein the endoscopic surgical device further includes a treatment tool (200) including a treatment tool insertion part (202) having a treatment part provided at a distal end thereof and an operating part (204) for operating the treatment part (206) provided at a base end of the treatment tool insertion part (202); and an overtube (300) that guides the endoscope insertion part (102) and the treatment tool insertion part (202) into a body cavity, in which the overtube (300) includes an overtube body (320) that passes through a body wall and is inserted into the body cavity, an endoscope insertion passage (306) that is provided inside the overtube body (320) and allows the endoscope insertion part (102) to be inserted therethrough so as to be movable forward and backward, and a treatment tool insertion passage (308) that is provided inside the overtube body (320) and allows the treatment tool insertion part (202) to be inserted therethrough so as to be movable forward and backward, **characterised in that** the endoscope (100) further comprises a hooking part (132) that is provided in the cable (104) and that is configured to hook a finger of a hand operating the operating part (204) to move the endoscope insertion part (102) forward and backward.

2. The endoscope (100) according to claim 1,
wherein the hooking part (132) includes an opening (134, 160) formed in a ring shape or in a C-shape, and the endoscope insertion part (102) is moved forward and backward by passing the finger through the opening (134, 160).

3. The endoscope (100) according to claim 1 or 2,
wherein the hooking part (132) is made of an elastic body.

4. The endoscope (100) according to any one of claims 1 to 3,
wherein a plurality of hooking parts (132) are provided in an axial direction of the cable (104).

5. The endoscope (100) according to any one of claims 1 to 4,
wherein the hooking part (132) is provided so as to be rotatable around an axis of the cable (104).

6. The endoscope (100) according to any one of claims 1 to 5,
wherein the hooking part (132) is provided so as to be attachable to and detachable from the cable (104).

7. The endoscope (100) according to any one of claims 1 to 6,
wherein the endoscope insertion part (102) is hard.

8. An endoscopic surgical device (10) comprising:
the endoscope (100) according to any one of claims 1 to 7; a treatment tool (200) as defined in claim 1; and an overtube (300) as defined in claim 1.

9. The endoscopic surgical device (10) according to claim 8, further comprising:
an interlocking member (400) including an endoscope-coupled part (420) coupled to the endoscope insertion part (102) inserted through the endoscope insertion passage (306) and a treatment tool-coupled part (422) coupled to the treatment tool insertion part (202) inserted through the treatment tool insertion passage (308) and being arranged inside the overtube body (320) so as to be movable forward and backward,
wherein the interlocking member (400) includes a dead zone where the forward and backward movement of either the endoscope insertion part (102) or the treatment tool insertion part (202) does not interlock with the movement of the other and a sensing zone where the forward and backward movement of either the endoscope insertion part (102) or the treatment tool insertion part (202) interlocks with the movement of the other.

## Patentansprüche

1. Endoskop (100), ausgebildet zur Verwendung für ein endoskopisches chirurgisches Gerät (10), welches das Endoskop (100) enthält, wobei das Endoskop aufweist: einen Endoskopeinführteil (102) mit einem Betrachtungsteil (116) an seinem distalen Ende, und mit einem flexiblen Kabel (104), welches an ein Basisende des Endoskopeinführteils (102) angeschlossen ist, wobei das endoskopische chirurgische Gerät weiterhin enthält: einen Behandlungswerkzeug (200) mit einem Behandlungswerkzeug-Einführteil (202) mit an dessen distalem Ende befindlichem Behandlungsteil, und mit einem Betätigungsteil (204) zum Betätigen des Behandlungsteils (206) an einem Basisende des Behandlungswerkzeug-Einführteils (202); und ein Überrohr (300), welches den Endoskopeinführteil (102) und den Behandlungswerkzeug-Einführteil (202) in einen Körperhohlraum leitet, wobei das Überrohr (300) einen Überrohrkörper (320) aufweist, der durch eine Körperwand hindurchgeht und in den Körperhohlraum eingeführt ist, einen Endoskopeinführkanal (306) im Inneren des Überrohrkörpers (320) zum Ermöglichen, dass der Endoskopeinführteil (102) durch ihn hindurch eingeführt wird, um vorwärts und rückwärts bewegt zu werden, und einen Behandlungswerkzeug-Einführkanal (308), der sich im Inneren des Überrohrköpers (320) befindet und dem Behandlungswerkzeug-Einführteil (202) ermöglicht, durch ihn hindurch eingeführt zu werden, um vorwärts und rückwärts bewegbar zu sein,
**dadurch gekennzeichnet, dass** das Endoskop (100) weiterhin einen Hakenteil (132) aufweist, der sich in dem Kabel (104) befindet und konfiguriert ist zum Einhaken eines Fingers einer Hand, die den Betätigungsteil (204) betätigt, um den Endoskopeinführteil (102) vorwärts und rückwärts zu bewegen.

2. Endoskop (100) nach Anspruch 1,
bei dem der Hakenteil (132) eine in Ringform oder C-Form ausgebildete Öffnung (134, 160) aufweist, und der Endoskopeinführteil (102) vorwärts und rückwärts dadurch bewegt wird, dass der Finger durch die Öffnung (134, 160) hindurchgreift.

3. Endoskop (100) nach Anspruch 1 oder 2, bei dem der Hakenteil (132) aus einem elastischen Körper gebildet ist.

4. Endoskop (100) nach einem der Ansprüche 1 bis 3, bei dem mehrere Hakenteile (132) in axialer Richtung des Kabels (104) vorgesehen sind.

5. Endoskop (100) nach einem der Ansprüche 1 bis 4, bei dem der Hakenteil (132) derart angeordnet ist, dass er um eine Achse des Kabels (104) drehbar ist.

6. Endoskop (100) nach einem der Ansprüche 1 bis 5, bei dem der Hakenteil (132) derart vorgesehen ist, dass er an dem Kabel (104) anbringbar und von diesem lösbar ist.

7. Endoskop (100) nach einem der Ansprüche 1 bis 6, bei dem der Endoskopeinführteil (102) hart ist.

8. Endoskopisches chirurgisches Gerät (10), umfassend:
das Endoskop (100) nach einem der Ansprüche 1 bis 7;
ein Behandlungswerkzeug (200) gemäß Anspruch 1; und
ein Überrohr (300) gemäß Anspruch 1.

9. Endoskopisches chirurgisches Gerät (10) nach Anspruch 8, weiterhin umfassend:
ein Sperrglied (400) mit einem an das Endoskop gekoppelten Teil (420), das mit dem Endoskopeinführteil (102) gekoppelt ist, das durch den Endoskopeinführkanal (306) eingeführt ist, und mit einem Behandlungswerkzeug-Kupplungsteil (402) gekoppelt ist, das mit dem Behandlungswerkzeug-Einführteil (202) gekoppelt ist, welches durch den Behandlungswerkzeug-Einführkanal (308) eingeführt ist, und sich im Inneren des Überrohrkörpers (320) befindet, um vorwärts und rückwärts bewegbar zu sein,
wobei das Sperrglied (400) eine Totzone enthält, in welcher die Vorwärts- und Rückwärtsbewegung entweder des Endoskopeinführteils (102) oder des Behandlungswerkzeug-Einführteils (202) sich nicht mit der Bewegung des anderen Teils verriegelt, und eine empfindliche Zone aufweist, in der die Vorwärts- und Rückwärtsbewegung entweder des Endoskopeinführteils (102) oder des Behandlungswerkzeug-Einführteils (202) sich mit der Bewegung des anderen Teils verriegelt.

## Revendications

1. Endoscope (100) apte à être utilisé pour un dispositif chirurgical endoscopique (10) incluant l'endoscope (100), dans lequel l'endoscope comprend une partie d'introduction d'endoscope (102) présentant une partie d'observation (116) prévue sur une extrémité distale de celle-ci et un câble flexible (104) raccordé à une extrémité de base de la partie d'introduction d'endoscope (102), et dans lequel le dispositif chirurgical endoscopique inclut en outre un outil de traitement (200) incluant une partie d'introduction d'outil de traitement (202) présentant une partie de traitement prévue sur une extrémité distale de celle-ci et une partie de manoeuvre (204) pour manoeuvrer la partie de traitement (206) prévue sur une extrémité de base de la partie d'introduction d'outil de traitement (202), et un surtube (300), lequel guide la partie d'introduction d'endoscope (102) et la partie d'introduction d'outil de traitement (202) dans une cavité corporelle, où le surtube (300) inclut un corps de surtube (320) lequel passe à travers une paroi corporelle et est introduit dans la cavité corporelle, un passage pour introduction d'endoscope (306), lequel est prévu dans le corps de surtube (320) et permet l'introduction de la partie d'introduction d'endoscope (102) à travers lui de manière à ce que celle-ci puisse se déplacer vers l'avant et l'arrière, et un passage pour introduction d'outil de traitement (308), lequel est prévu dans le corps de surtube (320) et permet l'introduction de la partie d'introduction d'outil de traitement (202) à travers lui de manière à ce que celle-ci puisse se déplacer vers l'avant et l'arrière,
**caractérisé en ce que** l'endoscope (100) comprend en outre une partie d'accrochage (132), laquelle est prévue dans le câble (104), et laquelle est configurée pour accrocher un doigt d'une main manoeuvrant la partie de manoeuvre (204) afin de déplacer la partie d'introduction d'endoscope (102) vers l'avant et l'arrière.

2. Endoscope (100) selon la revendication 1,
dans lequel la partie d'accrochage (132) inclut une ouverture (134, 160) façonnée en forme d'anneau ou en forme de C, et la partie d'introduction d'endoscope (102) est déplacée vers l'avant et l'arrière en passant le doigt à travers l'ouverture (134, 160).

3. Endoscope (100) selon la revendication 1 ou 2,
dans lequel la partie d'accrochage (132) est fabriquée à partir d'un corps élastique.

4. Endoscope (100) selon l'une quelconque des revendications 1 à 3,
dans lequel une pluralité de parties d'accrochage (132) sont prévues dans une direction axiale du câble (104).

5. Endoscope (100) selon l'une quelconque des revendications 1 à 4,
dans lequel la partie d'accrochage (132) est prévue de manière à pouvoir tourner autour d'un axe du câble (104).

6. Endoscope (100) selon l'une quelconque des revendications 1 à 5,
dans lequel la partie d'accrochage (132) est prévue de manière à pouvoir être attachée et détachée du câble (104).

7. Endoscope (100) selon l'une quelconque des revendications 1 à 6,
dans lequel partie d'introduction d'endoscope (102) est dure.

8. Dispositif chirurgical endoscopique (10), comprenant :
l'endoscope (100) selon l'une quelconque des revendications 1 à 7,
un outil de traitement (200) selon la revendication 1, et
un surtube (300) selon la revendication 1.

9. Dispositif chirurgical endoscopique (10) selon la revendication 8, comprenant en outre :
un élément de verrouillage réciproque (400) incluant une partie couplée à l'endoscope (420) couplée à la partie d'introduction d'endoscope (102) introduite à travers le passage pour introduction d'endoscope (306) et une partie couplée à l'outil de traitement (422) couplée à la partie d'introduction d'outil de traitement (202) introduite à travers le passage pour introduction d'outil de traitement (308) et agencée dans le corps de surtube (320) de manière à pouvoir être déplacée vers l'avant et vers l'arrière,
dans lequel l'élément de verrouillage réciproque (400) inclut une zone morte où le déplacement vers l'avant et vers l'arrière d'un élément parmi la partie d'introduction d'endoscope (102) ou la partie d'introduction d'outil de traitement (202) ne se verrouille pas sur le déplacement de l'autre élément, et une zone de détection où le déplacement vers l'avant et vers l'arrière soit de la partie d'introduction d'endoscope (102) soit de la partie d'introduction d'outil de traitement (202) se verrouille sur le déplacement de l'autre élément.
